# EUROPEAN PATENT SPECIFICATION

(11) **EP 3 472 317 B1**
(45) Date of publication and mention of the grant of the patent: **16.03.2022**
(21) Application number: 17814228.7
(22) Date of filing: 16.06.2017
(51) Int. Cl.: C12N 15/09, C12N 15/86, C12N 15/861, C07K 14/705, C07K 14/005, A61K 38/17, A61F 9/008, A61K 39/395, A61K 48/00, A61P 27/02

(54) **COMPOSITIONS AND METHODS FOR REDUCING OCULAR NEOVASCULARIZATION**
ZUSAMMENSETZUNGEN UND VERFAHREN ZUR VERRINGERUNG VON OKULARER NEOVASKULARISATION
COMPOSITIONS ET MÉTHODES DESTINÉES À RÉDUIRE LA NÉOVASCULARISATION OCULAIRE

(30) Priority: 16.06.2016 US 201662351231 P
(43) Date of publication of application: 24.04.2019
(60) Divisional application: 22154859.7
(73) Proprietor: Adverum Biotechnologies, Inc., Redwood City, CA 94063 (US)
(72) Inventor: BLUMENKRANZ, Mark, Menlo Park, CA 94025 (US); GASMI, Mehdi, Menlo Park, CA 94025 (US)
(74) Representative: EIP
(86) International application number: PCT/US2017/038012
(87) International publication number: WO 2017/218981

(56) References cited:
- WO-A2-2013/173129
- US-A1- 2011 200 530
- US-A1- 2014 242 031
- US-A1- 2014 294 771
- US-A1- 2015 259 395
- RUSLAN GRISHANIN ET AL: "Preclinical Evaluation of ADVM-022, a Novel Gene Therapy Approach to Treating Wet Age-Related Macular Degeneration", MOLECULAR THERAPY, vol. 27, no. 1, 1 January 2019 (2019-01-01), pages 118-129, XP055643284, ISSN: 1525-0016, DOI: 10.1016/j.ymthe.2018.11.003
- Anne M. Douar ET AL: "268. Optogenetic Engineering of Retinal Ganglion Cells with AAV2.7m8-ChrimsonRtdTomato (GS030-DP) Is Well Tolerated and Induces Functional Responses to Light in NonHuman Primates", Molecular Therapy Volume Gene & Cell Therapy S106 PNAS, 1 January 2013 (2013-01-01), XP055643766, Retrieved from the Internet: URL:https://www.cell.com/action/showPdf?pi i=S1525-0016(16)33077-5 [retrieved on 2019-11-19]
- Deniz Dalkara ET AL: "In vivo-directed evolution of a new adeno-associated virus for therapeutic outer retinal gene delivery from the vitreous", Science translational medicine, 12 June 2013 (2013-06-12), page 189ra76, XP055452295, United States Retrieved from the Internet: URL:http://stm.sciencemag.org/content/5/18 9/189ra76.full.pdf
- Anonymous: "History of Changes for Study: NCT01024998", , 5 January 2016 (2016-01-05), XP055643562, Retrieved from the Internet: URL:https://clinicaltrials.gov/ct2/history /NCT01024998?V_19=View#StudyPageTop [retrieved on 2019-11-18]
- TOBIAS WIMMER ET AL: "Functional Characterization of AAV-Expressed Recombinant Anti-VEGF Single-Chain Variable Fragments In Vitro", JOURNAL OF OCULAR PHARMACOLOGY AND THERAPEUTICS., vol. 31, no. 5, 1 June 2015 (2015-06-01), pages 269-276, XP055629564, US ISSN: 1080-7683, DOI: 10.1089/jop.2014.0125
- CHRISTIAN SCHÖN ET AL: "Retinal gene delivery by adeno-associated virus (AAV) vectors: Strategies and applications", EUROPEAN JOURNAL OF PHARMACEUTICS AND BIOPHARMACEUTICS., vol. 95, 1 September 2015 (2015-09-01), pages 343-352, XP055533708, NL ISSN: 0939-6411, DOI: 10.1016/j.ejpb.2015.01.009
- MICHAEL JOHN TOLENTINO ET AL: "Drugs in Phase II clinical trials for the treatment of age-related macular degeneration", EXPERT OPINION ON INVESTIGATIONAL DRUGS, vol. 24, no. 2, 22 September 2014 (2014-09-22), pages 183-199, XP55263180, UK ISSN: 1354-3784, DOI: 10.1517/13543784.2015.961601
- NORK, TM et al.: "Prevention of Experimental Choroidal Neovascularization and Resolution of Active Lesions by VEGF Trap in Nonhuman Primates", Archives of Ophthalmology, vol. 129, no. 8, August 2011 (2011-08), pages 1042-1052, XP055138977, DOI: doi:10.1001/archophthalmol.2011.210
- Anonymous: "Application for inclusion in the WHO Essential Medicines List, Section 21 Ophthalmological Preparations Ranibizumab (Lucentis(R)) - Addition", NOVARTIS, 28 November 2014 (2014-11-28), page 42, XP055600842,
- COSTA, RA et al.: "Intravitreal Bevacizumab for Choroidal Neovascularization Caused by AMD (IBeNA Study): Results of a Phase 1 Dose-Escalation Study", Investigative Ophthalmology & Visual Science, vol. 47, no. 10, October 2006 (2006-10), pages 4569-4578, XP055600846, ISSN: 1552-5783, DOI: 10.1167/iovs.06-0433

## Description

### BACKGROUND OF THE DISCLOSURE

Vascular endothelial growth factor (VEGF) is a signal protein produced by cells that stimulates vasculogenesis and angiogenesis. VEGF can be a part of the system that restores the oxygen supply to tissues when blood circulation is inadequate. The normal function of VEGF can be to create new blood vessels during embryonic development, new blood vessels after injury, muscle following exercise, and new vessels to bypass blocked vessels.

Overexpression of VEGF can contribute to various disease states and conditions in mammals. Expression of VEGF in certain cancers can allow the cancer cells to grow and metastasize. Overexpression of VEGF can cause vascular disease in the retina of the eye and other parts of the body.

VEGF and VEGF receptors (VEGFRs) are implicated in a number of diseases, including the development of choroidal neovascularization (CNV) and age-related macular degeneration. Examples of eye diseases or conditions associated with VEGF and/or VEGFR activity include neovascular (wet) age-related macular degeneration (AMD), macular edema following retinal vein occlusion (RVO), diabetic macular edema (DME), diabetic retinopathy (DR) in patients with DME, ischemic retinopathy, intraocular neovascularization, dry-AMD, retinal neovascularization, diabetic retina ischemia, diabetic retinal edema, proliferative diabetic retinopathy, central retinal vein occlusion, and branched retinal vein occlusion.

Wimmer et al. (2015) "Functional Characterization of AAV-Expressed Recombinant Anti-VEGF Single-Chain Variable Fragments In Vitro", JOURNAL OF OCULAR PHARMACOLOGY AND THERAPEUTICS vol. 31, no. 5, 1, pages 269-276 report the results of *in vitro* experiments that were performed to determine whether anti-VEGF molecules derived from ranibizumab can be produced in a mammalian kidney cell line following AAV-mediated gene transfer.

### SUMMARY OF THE DISCLOSURE

The invention provides pharmaceutical compositions for use as defined in the appended claims. The disclosure set out below provides technical background information and is not intended to define the invention as such, but rather to facilitate the understanding and working of the invention while placing it in technical context.

While some protein- or antibody-based injection therapies are available for the treatment of AMD, e.g., ranibizumab and bevacizumab, a gene therapy method of delivering an anti-VEGF agent into an eye can provide an improved treatment option for patients because gene therapy can provide prolonged or sustained release of the therapeutic agent in vivo without requiring repeated injections, which can increase the risks of inflammation, infection, and other adverse effects in some patients. Additionally, by not requiring repeated injections, gene therapy addresses the patient compliance and adherence challenge associated with therapies that require repeated injections, as non-compliance can result in vision loss and deterioration of the eye disease or condition. The rate of non-compliance and non-adherence to treatment regimens that require repeated or frequent trips to medical offices for administration is higher among elderly patients, who are most impacted by AMD. Delivering a therapeutic agent into an eye of a patient via gene therapy can thus provide a safer, potentially more cost-effective, and more convenient treatment option for patients, and improve patient outcomes by addressing the non-compliance and non-adherence problem.

The present disclosure relates to pharmaceutical compositions and methods of prevention or treatment of ocular neovascularization, such as AMD and CNV, in a subject (e.g., a human subject) by administering subretinally or intravitreally a pharmaceutical composition comprising a pharmaceutically effective amount of a vector or viral particles comprising a nucleic acid encoding an anti-VEGF agent, such as sFlt-1, ranibizumab, or bevacizumab.

In some aspects, disclosed herein is a method of treating an eye disease or condition, the method comprising administering a unit dose of a pharmaceutical suspension to a primate subject by injection to an eye, wherein a unit dose of the pharmaceutical suspension comprises: between 1E12 to 1E13 vector genomes of rAAV having a variant capsid protein comprising an insertion of amino acid sequence selected from LGETTRP, NETITRP, KAGQANN, KDPKTTN, KDTDTTR, RAGGSVG, AVDTTKF, and STGKVPN at a position that corresponds to amino acids 570-611 of capsid protein VP1 in AAV2; and a heterologous sequence encoding an anti-vascular endothelial growth factor (anti-VEGF) polypeptide. In some cases, the unit dose comprises between 2E12 to 6E12 vector genomes. In some cases, the subject is a non-human primate. In some cases, the subject is a human. In some cases, the eye condition or disease is neovascular (wet) age-related macular degeneration (AMD), macular edema following retinal vein occlusion, diabetic macular edema (DME), retinal vein occlusion, or diabetic retinopathy associated with DME. In some cases, the eye condition or disease is choroidal neovascularization or AMD. In some cases, administering the suspension results in a reduction in percentage of grade IV lesions by at least 5% as compared to a vehicle control, as measured by color fundus photography. In some cases, the reduction in percentage of grade IV lesions is at least 10%. In some cases, the unit dose comprises a volume that is not more than 100 µL. In some cases, the unit dose comprises a volume that is not more than 50 µL. In some cases, the insertion is LGETTRP at a position between amino acids 587 and 588 in AAV2. In some cases, the subject is responsive to at least one of ranibizumab, bevacizumab, and sVEGFR-1. In some cases, the subject has been pre-treated with ranibizumab or bevacizumab. In some instances, the injection is intravitreal. In some instances, the injection is subretinal. In some cases, the administering by injection occurs not more than once in at least 2 years. In some cases, the administering by injection occurs not more than once in at least 5 years. In some cases, the administering is a one-time administration. In some cases, the method further comprises agitating the suspension to ensure even distribution prior to the administering step. In some cases, the method further comprises warming the suspension to room temperature prior to the administering step. In some cases, the suspension further comprises a surfactant. In some cases, the surfactant is selected from polysorbates, sodium dodecyl sulfate, sodium lauryl sulfate, lauryl dimethyl amine oxide, polyethoxylated alcohols, polyoxyethylene sorbitan, octoxynol, Brij, pluronic, and polyoxyl castor oil. In some cases, the suspension further comprises phenol, mannitol, sorbitol, or sodium chloride. In some cases, the method further comprises administering an antibiotic solution or an atropine sulfate ointment after the injection. In some cases, the antibiotic solution comprises ciprofloxacin. In some cases, the anti-VEGF polypeptide is a humanized monoclonal antibody. In some cases, the anti-VEGF polypeptide is an antibody fragment or Fab. In some cases, the humanized monoclonal antibody is ranibizumab or bevacizumab. In some cases, the anti-VEGF polypeptide is a soluble, truncated form of VEGF receptor 1 (sVEGFR-1).

In other aspects, also disclosed herein is a method of treating an eye condition or disease, the method comprising: agitating a suspension composition, comprising: a rAAV having a variant capsid protein comprising an insertion of amino acid sequence selected from LGETTRP, NETITRP, KAGQANN, KDPKTTN, KDTDTTR, RAGGSVG, AVDTTKF, and STGKVPN at a position that corresponds to amino acids 570-611 of capsid protein VP1 in AAV2; and a heterologous sequence encoding an anti-vascular endothelial growth factor (anti-VEGF) polypeptide; and administering the suspension composition to an eye of a human subject via injection. In some cases, the insertion is LGETTRP between amino acids 587 and 588 of AAV2. In some cases, the subject is characterized as having been pre-treated with ranibizumab or bevacizumab. In some cases, the subject is responsive to at least one of ranibizumab and bevacizumab. In some cases, the anti-VEGF polypeptide is a humanized monoclonal antibody. In some cases, the anti-VEGF polypeptide is an antibody fragment or Fab. In some cases, the humanized monoclonal antibody is ranibizumab or bevacizumab. In some cases, the anti-VEGF polypeptide is a soluble, truncated form of VEGF receptor 1 (sVEGFR-1). In some cases, the volume administered to the subject is not more than 50 µL. In some cases, the volume administered to the subject is not more than 100 µL. In some cases, the volume comprises a unit dose of between 1E12 to 1E13 vector genomes. In some cases, the volume comprises a unit dose of between 2E12 to 6E12 vector genomes. In some case, the administering step occurs not more than once in at least 2 years. In some cases, the administering step is a one-time injection. In some cases, the method further comprises assaying the subject for responsiveness to at least one approved therapy before administering the composition. In some cases, the approved therapy comprises ranibizumab and bevacizumab. In some cases, the suspension comprises a pharmaceutically acceptable excipient. In some cases, the excipient comprises a surfactant or a stabilizer. In some cases, the surfactant is selected from polysorbates, sodium dodecyl sulfate, sodium lauryl sulfate, lauryl dimethyl amine oxide, polyethoxylated alcohols, polyoxyethylene sorbitan, octoxynol, Brij, pluronic, and polyoxyl castor oil. In some cases, the pharmaceutically acceptable excipient comprises phenol, mannitol, sorbitol, or sodium chloride. In some cases, the eye condition or disease is neovascular (wet) age-related macular degeneration (AMD), macular edema following retinal vein occlusion, diabetic macular edema (DME), retinal vein occlusion, or diabetic retinopathy associated with DME. In some cases, the eye condition or disease is choroidal neovascularization or AMD. In some cases, the injection is intravitreal. In some instances, the injection is subretinal. In some cases, the insertion is LGETTRP at a position between amino acids 587 and 588 in AAV2. In some cases, the method further comprises warming the suspension to room temperature before administering.

In other aspects, also disclosed herein is a pharmaceutical composition comprising a unit dose of a suspension, comprising: a rAAV having a variant capsid protein comprising an insertion of amino acid sequence selected from LGETTRP, NETITRP, KAGQANN, KDPKTTN, KDTDTTR, RAGGSVG, AVDTTKF, and STGKVPN at a position that corresponds to amino acids 570-611 of capsid protein VP1 in AAV2; and a heterologous sequence encoding an anti-vascular endothelial growth factor (anti-VEGF) polypeptide. In some cases, the unit dose is between 1E12 to1E13 vector genomes. In some cases, the unit dose is between 2E12 to 6E12 vector genomes. In some cases, the suspension is refrigerated. In some cases, a kit comprises the pharmaceutical composition and a solution for diluting the pharmaceutical composition. In some cases, the solution comprises a buffer, salt, alcohol, a surfactant, or any combination thereof. In some cases, the kit further comprises a syringe. In some cases, the anti-VEGF polypeptide is a humanized monoclonal antibody. In some cases, the anti-VEGF polypeptide is an antibody fragment or Fab. In some cases, the humanized monoclonal antibody is ranibizumab or bevacizumab. In some cases, the anti-VEGF polypeptide is a soluble, truncated form of VEGF receptor 1 (sVEGFR-1). In some cases, the insertion is LGETTRP at a position between amino acids 587 and 588 in AAV2.

### BRIEF DESCRIPTION OF THE DRAWINGS

A better understanding of the features and advantages of the present invention will be obtained by reference to the following detailed description, in which the principles of the invention are utilized, and the accompanying drawings of which:

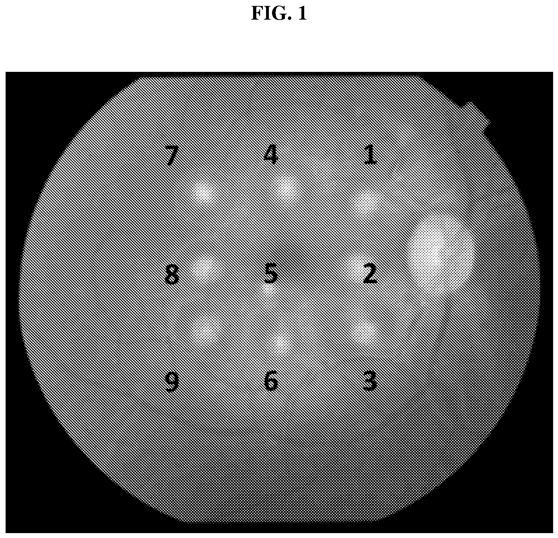

**FIG. 1** illustrates laser choroidal neovascularization (CNV) in a non-human primate model (African green monkeys). Nine lesions were induced by single laser application using laser irradiation of 750 mW, 50 µm, 100 ms for all spots except the central spot, which was treated with 400 mW. Color fundus photography was performed immediately after the laser treatment to document the laser lesions.

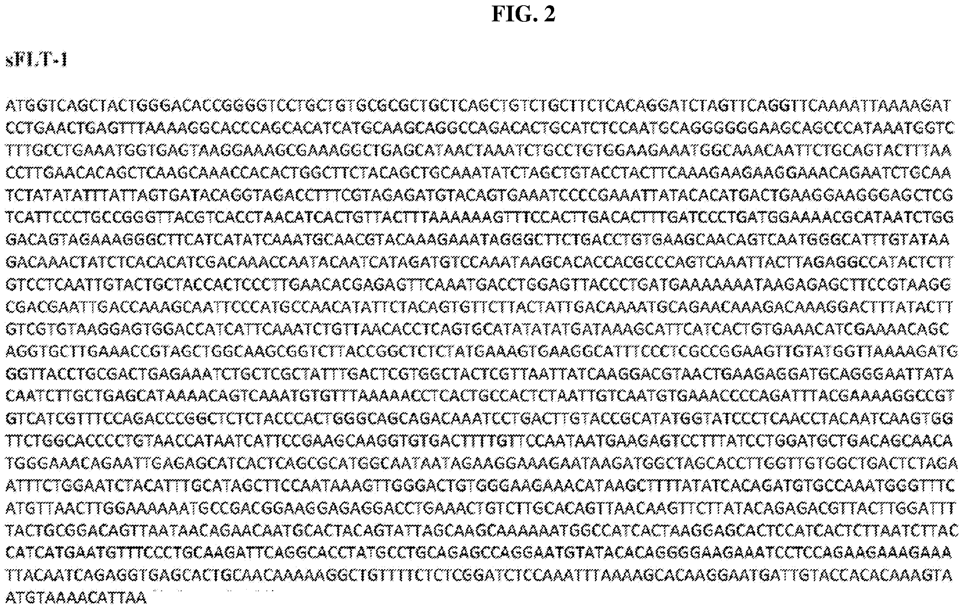

**FIG. 2** illustrates the nucleic acid sequence of sVEGFR-1.

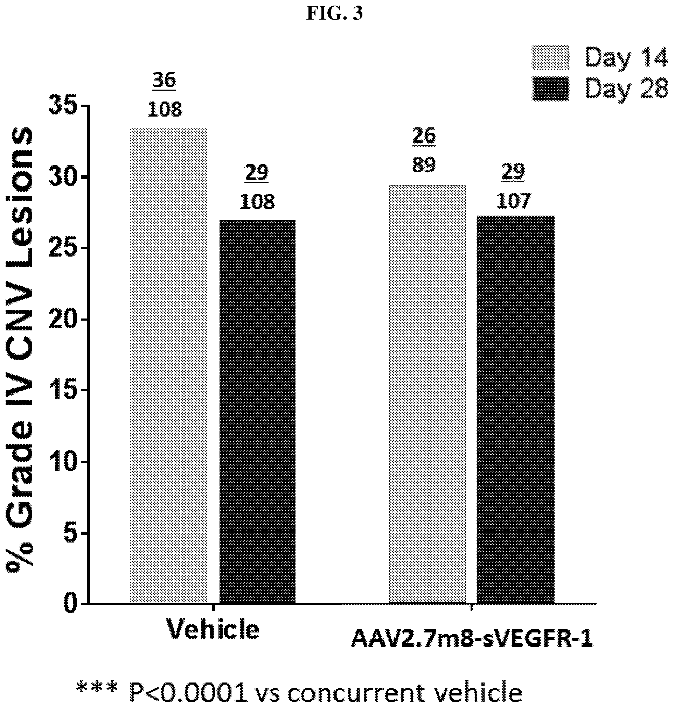

**FIG. 3** illustrates CNV reduction after intravitreal injection of AAV2.7m8-sVEGFR-1. AAV2.7m8-sVEGFR-1 or a vehicle control comprising formulation buffer was administered to eyes of monkeys via intravitreal injection at a dose of 2.1 × 10¹² vg. A decrease in the percent grade IV CNV lesions was observed for AAV2.7m8-sVEGFR-1 as compared to administration of vehicle alone for the fundus image collected at day 14 (light gray bar). No significant difference in the percent grade IV CNV lesions was observed for AAV2.7m8-sVEGFR-1 as compared to administration of vehicle alone as measured by fundus images collected at day 28 (dark gray bar).

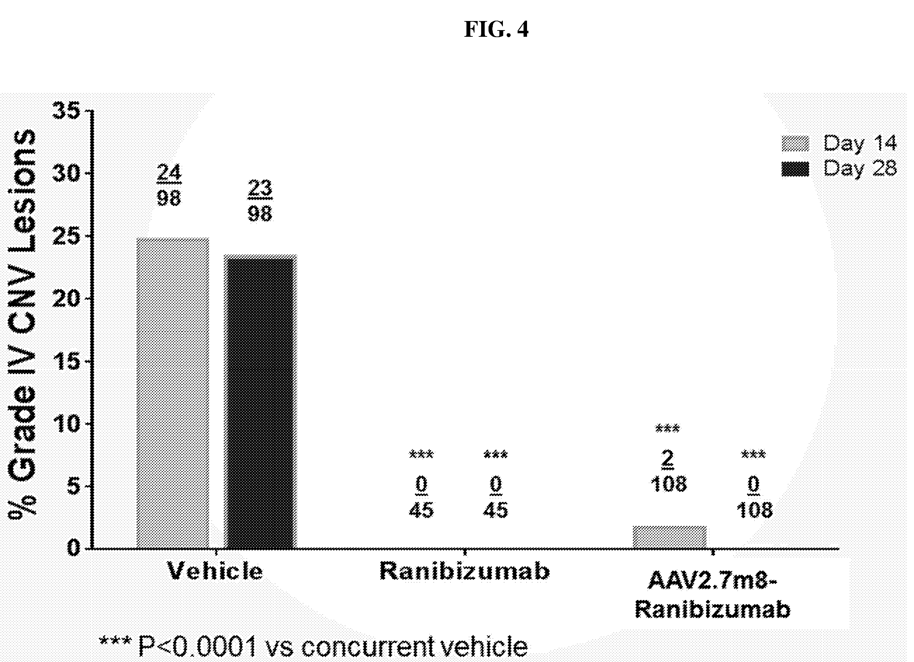

**FIG. 4** illustrates AAV2.7m8-ranibizumab administered intravitreally prevented the occurrence of laser-induced grade IV CNV lesions. AAV2.7m8-ranibizumab, ranibizumab alone (positive control), or vehicle control comprising formulation buffer were administered to eyes of monkeys via intravitreal injection at a dose of 2 × 10¹² vg. AAV2.7m8-ranibizumab significantly reduced grade IV CNV lesions to levels comparable to ranibizumab alone as measured by fundus images collected at day 14 (light gray bar) and day 28 (dark gray bar).

### DETAILED DESCRIPTION OF THE DISCLOSURE

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

The present disclosure relates to pharmaceutical compositions and methods of treatment or prevention of eye diseases or conditions comprising administering a gene therapy, a vector, or a construct by intravitreal or subretinal injection into an eye of a primate (e.g., a monkey or a human) comprising a nucleic acid sequence (e.g., cDNA) that encodes an anti-VEGF agent. Upon intravitreal or subretinal injection of a gene therapy, a vector, or a construct, comprising a nucleic acid sequence that encodes an anti-VEGF agent or transgene, the anti-VEGF gene is expressed in vivo in target cells or tissue, e.g., in retinal cells, to generate anti-VEGF protein or gene product to produce a therapeutic effect.

In some cases, a gene therapy, vector, or construct comprising an anti-VEGF agent is used to treat or prevent one or more eye diseases or conditions, including, but not limited to, neovascular (wet) age-related macular degeneration (AMD), retinal vein occlusion (RVO), macular edema following RVO, diabetic macular edema (DME), and/or diabetic retinopathy (DR) in patients with DME, or any other related eye disease or condition involving neovascularization (e.g., choroidal neovascularization (CNV)) in a primate or human subject. In some cases, methods described herein are used to treat an eye disease or condition that is responsive to a standard of care therapy or an existing treatment, e.g., ranibizumab or bevacizumab injection. In some cases, methods described herein are used to treat an eye disease or condition that is responsive to at least one current standard of care, e.g., ranibizumab or bevacizumab injection, for AMD, RVO, DME, DR, or DR in patients with DME

The present disclosure relates to compositions and methods for the prevention or treatment of ocular neovascularization in a subject (e.g., non-human primate or human), by administering either subretinally or intravitreally a pharmaceutical composition adapted for gene therapy, comprising a pharmaceutically effective amount of a vector, e.g., a viral vector such as adeno-associated virus (AAV), comprising a nucleic acid encoding an anti-VEGF agent, a therapeutic transgene, or a nucleic acid sequence that encodes a polypeptide having at least 75%, 80%, 81%, 82%, 83%, 84%, 85%, 86%, 87%, 88%, 89%, 90%, 91%, 92%, 93%, 94%, 95%, 96%, 97%, 98%, 99%, or 100% homology to sVEGFR-1, ranibizumab, bevacizumab, or any other known ant-VEGF agent, or any functional fragment, mutant, or variant thereof. Such homology can be based on the nucleic acid sequence (e.g., cDNA), amino acid sequence, spatial conformation, or protein structure (e.g., secondary, tertiary, or quaternary structure).

In some aspects, a vector disclosed herein is an adeno-associated virus (AAV) of any serotype, comprising a mutation, such as an insertion of 5 to 11 amino acids at a site in the solvent-exposed GH loop or loop IV of a capsid protein. In some cases, a 7-mer amino acid sequence is inserted in the GH loop or loop IV of an AAV capsid protein. For the GH loop/loop IV of AAV capsid, see, e.g., van Vliet et al. (2006) Mol. Ther. 14:809; Padron et al. (2005) J. Virol. 79:5047; and Shen et al. (2007) Mol. Ther. 15:1955. In some cases, an amino acid sequence comprising any one of the following: LGETTRP, NETITRP, KAGQANN, KDPKTTN, KDTDTR, RAGGSVG, AVDTTKF, and STGKVPN is inserted in the GH loop/loop IV of AAV capsid protein (e.g., VP1 capsid protein), thus creating AAV variants, each having a variant capsid protein. In some cases, the amino acid insertion occurs at the following positions in each AAV serotype: between 587 and 588 of AAV2, between amino acids 590 and 591 of AAV1, between amino acids 575 and 576 of AAV5, between amino acids 590 and 591 of AAV6, between amino acids 589 and 590 of AAV7, between amino acids 590 and 591 of AAV8, between amino acids 588 and 589 of AAV9, or between amino acids 589 and 590 of AAV10.

In some cases, amino acids can be inserted between two adjacent amino acids at a position between amino acids 570 and 611 of VP1 of AAV2 or the corresponding position in the capsid protein of another AAV serotype. In some cases, an AAV2 vector comprising LGETTRP amino acid insertion between amino acids 587 and 588 of VP1 of AAV2 is used for gene therapy disclosed herein. In some cases, methods of treatment as described herein comprise administering subretinally or intravitreally a pharmaceutical composition or formulation comprising an AAV of any serotype comprising a nucleic acid sequence encoding an anti-VEGF agent (e.g., sVEGFR-1, ranibizumab, or bevacizumab). In some aspects, subretinal or intravitreal injection of the pharmaceutical compositions disclosed herein results in an expression of the anti-VEGF agent in target cells in an eye of a subject, e.g., retinal cells, which results in a reduction of neovascularization or VEGF expression and/or inhibition of VEGF expression or activity in vivo, or disruption of VEGF-VEGFR interaction in vivo. In some instances, expression of the anti-VEGF agent sequesters endogenous VEGF in vivo to prevent VEGF binding or interaction with endogenous VEGF receptors in vivo.

One advantage of gene therapy over protein injections is that gene therapy provides for prolonged or continued release of a therapeutic agent (e.g., anti-VEGF agent) and does not require repeated injections. This prolonged or sustained release of the therapeutic agent results from the delivery of a nucleic acid sequence that encodes the transgene, which is expressed in vivo to provide a therapeutic effect.

In some cases, a rAAV can comprise a capsid variant protein that increases its infectivity of the target cells or tissue in an eye (e.g., retinal cells), allowing more efficient delivery of the nucleic acid sequence encoding a therapeutic transgene into the target cells or tissue where the therapeutic transgene can be expressed over a period of time, e.g., at least 1, 1.5, 2, 3, 4, 5, 10, or more years. Gene therapy as disclosed herein can target a specific tissue or cell type of interest, e.g., photoreceptor cells, which can help to minimize off-target effects, or provide a more targeted delivery of the therapeutic transgene in vivo.

With prolonged or sustained delivery of an anti-VEGF agent in vivo via gene therapy, one would be able to administer the pharmaceutical composition comprising a nucleic acid sequence that encodes the anti-VEGF agent, in a single dose or a one-time dose. In some cases, the total number of doses of a gene therapy administered to a subject is not more than once in at least 1.5 years, in at least 2 years, at least 3 years, at least 4 years, at least 5 years, at least 6 years, at least 7 years, at least 8 years, at least 9 years, or at least 10 years. In some cases, administration of a gene therapy comprising a nucleic acid sequence encoding an anti-VEGF agent is only one time or once in the life of a patient. In some cases, one-time administration of a gene therapy comprising a nucleic acid sequence encoding an anti-VEGF agent can produce a therapeutic effect in a patient that lasts for more than 1 year, or for more than 2, 3, 4, 5, 6, 7, 8, 9, 10 or more years. In some cases, a gene therapy comprising a nucleic acid sequence encoding an anti-VEGF agent is administered not more than once to a patient in at least 2 or more, at least 3 or more, at least 4 or more, at least 5 or more, at least 6 or more, at least 7 or more, at least 8 or more, at least 9 or more, or at least 10 or more years. In some cases, a gene therapy comprising a nucleic acid sequence encoding an anti-VEGF agent is administered to a patient who is responsive to at least one current standard of care or at least one existing therapy, e.g., ranibizumab or bevacizumab. In some cases, the gene therapy is administered to patients who received a pre-treatment with ranibizumab or bevacizumab before receiving the gene therapy.

In some cases, the one-time administration of a gene therapy comprising a nucleic acid sequence encoding an anti-VEGF agent obviates the need for the patient to receive ranibizumab, bevacizumab, or any other protein-based therapeutics or standard of care treatments for neovascularization in the eye for more than a year, for more than 1.5 years, or for more than 2, 3, 4, 5, 6, 7, 8, 9, 10 years. In some cases, a patient who receives an injection of a gene therapy comprising a nucleic acid sequence encoding an anti-VEGF agent does not need any additional injections of ranibizumab, bevacizumab, or any other protein-based therapeutics or standard of care treatments for neovascularization in the eye for the remainder of the patient's life. In other cases, a patient who receives a one-time injection of an anti-VEGF gene therapy can commence therapy with ranibizumab, bevacizumab, and/or any other approved therapeutics, as needed, after at least 1.5, 2, 5, 10 or more years have lapsed after receiving the gene therapy.

The terminology of the present disclosure is for the purpose of describing particular cases only and is not intended to be limiting of compositions, methods and compositions of this disclosure.

The compositions and methods of this disclosure as described herein may employ, unless otherwise indicated, conventional techniques and descriptions of molecular biology (including recombinant techniques), cell biology, biochemistry, immunochemistry and ophthalmic techniques, which are within the skill of those who practice in the art. Such conventional techniques include methods for observing and analyzing the retina, or vision in a subject, cloning and propagation of recombinant virus, formulation of a pharmaceutical composition, and biochemical purification and immunochemistry. Specific illustrations of suitable techniques can be had by reference to the examples herein. However, equivalent conventional procedures can, of course, also be used. Such conventional techniques and descriptions can be found in standard laboratory manuals such as Green, et al., Eds., Genome Analysis: A Laboratory Manual Series (Vols. I-IV) (1999); Weiner, et al., Eds., Genetic Variation: A Laboratory Manual (2007); Dieffenbach, Dveksler, Eds., PCR Primer: A Laboratory Manual (2003); Bowtell and Sambrook, DNA Microarrays: A Molecular Cloning Manual (2003); Mount, Bioinformatics: Sequence and Genome Analysis (2004); Sambrook and Russell, Condensed Protocols from Molecular Cloning: A Laboratory Manual (2006); and Sambrook and Russell, Molecular Cloning: A Laboratory Manual (2002) (all from Cold Spring Harbor Laboratory Press); Stryer, L., Biochemistry (4th Ed.) W.H. Freeman, N.Y. (1995); Gait, "Oligonucleotide Synthesis: A Practical Approach" IRL Press, London (1984); Nelson and Cox, Lehninger, Principles of Biochemistry, 3rd Ed., W.H. Freeman Pub., New York (2000); and Berg et al., Biochemistry, 5th Ed., W.H. Freeman Pub., New York (2002).

In some cases, disclosed herein are pharmaceutical formulations comprising: (a) a recombinant adeno-associated virus (rAAV2) virion adapted for gene therapy comprising: (i) a variant AAV2 capsid protein, wherein the variant AAV2 capsid protein comprises LGETTRP insertion between positions 587 and 588, and wherein the variant capsid protein confers an increase in an infectivity of retinal cells relative to an AAV virion that comprises a corresponding non-variant AAV2 capsid protein; and (ii) a heterologous nucleic acid sequence encoding an anti-VEGF agent; and (b) a pharmaceutically acceptable excipient. In some cases, the gene product that is encoded is a polypeptide having at least 75%, 80%, 81%, 82%, 83%, 84%, 85%, 86%, 87%, 88%, 89%, 90%, 91%, 92%, 93%, 94%, 95%, 96%, 97%, 98%, 99%, or 100% homology to ranibizumab, bevacizumab, or any other known anti-VEGF agent.

Also disclosed herein are methods of treating an eye condition or disease for which the anti-VEGF gene product (e.g., ranibizumab or bevacizumab) is indicated or approved for treating, comprising administering a pharmaceutical composition adapted for gene therapy, i.e., delivering a nucleic acid sequence that encodes an anti-VEGF gene product in vivo, as described herein, to an eye of a subject by subretinal or intravitreal injection. In some cases, the gene therapy is administered by intravitreal injection. In some cases, the anti-VEGF agent is ranibizumab, bevacizumab, sVEGFR-1, or any variant or functional fragment thereof.

Also disclosed herein are pharmaceutical compositions comprising a gene therapy or a vector that encodes a fusion protein or polypeptide having at least 75%, 80%, 81%, 82%, 83%, 84%, 85%, 86%, 87%, 88%, 89%, 90%, 91%, 92%, 93%, 94%, 95%, 96%, 97%, 98%, 99%, or 100% homology to a known anti-VEGF protein or fusion protein (e.g., ranibizumab or bevacizumab), wherein the pharmaceutical compositions can be lyophilized, or supplied in lyophilized form. In some cases, a lyophilized form of the pharmaceutical composition is provided in a kit with a solution or buffer for reconstituting the pharmaceutical composition before administration. In some cases, the pharmaceutical compositions disclosed herein are supplied as a solution, a homogeneous solution, a suspension, or a refrigerated suspension.

Also disclosed herein are recombinant adeno-associated virus (rAAV) virions adapted for gene therapy for reducing choroidal neovascularization comprising: (a) a variant AAV capsid protein, wherein the variant capsid protein confers an increase in an infectivity of retinal cells relative to an AAV virion that comprises a corresponding non-variant or unmodified AAV capsid protein; (b) a heterologous nucleic acid sequence encoding a polypeptide or therapeutic transgene with anti-VEGF activity. In some cases, the rAAV used for gene therapy is rAAV2.

Also disclosed herein are methods of treating an eye condition or disease comprising administering a rAAV virion adapted for gene therapy and in vivo delivery of a nucleic acid sequence for expressing an anti-VEGF agent, or a protein having an anti-VEGF activity, as described herein to an eye of a human subject; where the human subject has been previously diagnosed with an eye condition associated with neovascularization. In some cases, the gene therapy is administered to a patient who is responsive to at least one of the approved anti-VEGF therapies, e.g., ranibizumab or bevacizumab. In some cases, the gene therapy is administered to a patient pre-treated with at least one of the approved therapies, e.g., ranibizumab or bevacizumab. In some cases, the gene therapy disclosed herein is administered to a patient who was pre-treated with at least one of the approved therapies, e.g., ranibizumab or bevacizumab injections, and failed to show improvement. In some cases, patients who receive the gene therapy disclosed herein have one or more risk factors that disfavor treating the patient with therapies that require multiple, repeated injections to an eye, e.g., increased risk of inflammation, infection, elevated intraocular pressure, and/or other adverse effects.

In some cases, disclosed herein are methods and pharmaceutical formulations comprising: (a) a recombinant adeno-associated virus (rAAV) virion adapted for gene therapy comprising: (i) a variant AAV capsid protein comprising an amino acid insertion selected from LGETTRP, NETITRP, KAGQANN, KDPKTTN, KDTDTTR, RAGGSVG, AVDTTKF, and STGKVPN at a position that corresponds to amino acids 570-611 of capsid protein VP1 in AAV2, and where the variant capsid protein confers an increase in an infectivity of a retinal cell relative to an AAV virion that comprises a corresponding non-variant AAV2 capsid protein; and (ii) a heterologous nucleic acid sequence encoding an anti-VEGF agent; and (b) a pharmaceutically acceptable excipient. In some cases, the gene product that is encoded is a fusion protein, antibody, or an antibody fragment. In some cases, a pharmaceutically acceptable excipient comprises a surfactant that prevents aggregation in the pharmaceutical composition disclosed herein.

Unless otherwise defined, all technical terms used herein have the same meaning as commonly understood by one of ordinary skill in the art.

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The term "comprising" as used herein is synonymous with "including" or "containing", and is inclusive or open-ended.

Any reference to "or" herein is intended to encompass "and/or" unless otherwise stated. As used herein, the term "about" a number refers to that number plus or minus 10% of that number. The term "about" a range refers to that range minus 10% of its lowest value and plus 10% of its greatest value.

The term "subject", "patient", or "individual" refers to primates, including non-human primates, e.g., African green monkeys and rhesus monkeys, and humans. In preferred cases, the subject is a human or a human patient.

The terms "treat," "treating", "treatment," "ameliorate" or "ameliorating" and other grammatical equivalents as used herein, include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and are intended to include prophylaxis. The terms further include achieving a therapeutic benefit and/or a prophylactic benefit. By therapeutic benefit is meant eradication or amelioration of the underlying disease being treated. Also, a therapeutic benefit is achieved with the eradication or amelioration of one or more of the physiological symptoms associated with the underlying disease such that an improvement is observed in the patient, notwithstanding that, in some cases, the patient is still afflicted with the underlying disease. For prophylactic benefit, the pharmaceutical compositions are administered to a patient at risk of developing a particular disease, or to a patient reporting one or more of the physiological symptoms of a disease, even if a diagnosis of the disease has not been made.

The terms "administer," "administering", "administration," and the like, as used herein, can refer to the methods that are used to enable delivery of therapeutics or pharmaceutical compositions to the desired site of biological action. These methods include intravitreal or subretinal injection to an eye.

The terms "effective amount", "therapeutically effective amount" or "pharmaceutically effective amount" as used herein, can refer to a sufficient amount of at least one pharmaceutical composition or compound being administered which will relieve to some extent one or more of the symptoms of the disease or condition being treated.

The term "pharmaceutically acceptable" as used herein, can refer to a material, such as a carrier or diluent, which does not abrogate the biological activity or properties of a compound disclosed herein, and is relatively nontoxic (i.e., when the material is administered to an individual it does not cause undesirable biological effects nor does it interact in a deleterious manner with any of the components of the composition in which it is contained).

The term "pharmaceutical composition," or simply "composition" as used herein, can refer to a biologically active compound, optionally mixed with at least one pharmaceutically acceptable chemical component, such as, though not limited to carriers, stabilizers, diluents, dispersing agents, suspending agents, thickening agents, excipients and the like.

An "AAV vector" or "rAAV vector" as used herein refers to an adeno-associated virus (AAV) vector or a recombinant AAV (rAAV) vector comprising a polynucleotide sequence not of AAV origin (i.e., a polynucleotide heterologous to AAV such as a nucleic acid sequence that encodes a therapeutic transgene, e.g., ranibizumab), typically a sequence of interest for the genetic transformation of a cell. In general, the heterologous polynucleotide is flanked by at least one, and generally by two, AAV inverted terminal repeat sequences (ITRs). The term rAAV vector encompasses both rAAV vector particles and rAAV vector plasmids. A rAAV vector may either be single-stranded (ssAAV) or self-complementary (scAAV).

An "AAV virus" or "AAV viral particle" or "rAAV vector particle" refers to a viral particle composed of at least one AAV capsid protein (typically by all of the capsid proteins of a wild-type AAV) and a polynucleotide rAAV vector. If the particle comprises a heterologous polynucleotide (i.e. a polynucleotide other than a wild-type AAV genome such as a transgene to be delivered to a mammalian cell), it is typically referred to as an "rAAV vector particle" or simply an "rAAV vector". Thus, production of rAAV particle necessarily includes production of rAAV vector, as such a vector is contained within a rAAV particle..

The term "packaging" as used herein can refer to a series of intracellular events that can result in the assembly and encapsidation of a rAAV particle.

AAV "rep" and "cap" genes refer to polynucleotide sequences encoding replication and encapsidation proteins of adeno-associated virus. AAV rep and cap are referred to herein as AAV "packaging genes."

The term "polypeptide" can encompass both naturally-occurring and non-naturally occurring proteins (e.g., a fusion protein), peptides, fragments, mutants, derivatives and analogs thereof. A polypeptide may be monomeric, dimeric, trimeric, or polymeric. Further, a polypeptide may comprise a number of different domains each of which has one or more distinct activities. For the avoidance of doubt, a "polypeptide" may be any length greater two amino acids.

As used herein, "polypeptide variant" or simply "variant" refers to a polypeptide whose sequence contains an amino acid modification. In some instances, the modification can be an insertion, duplication, deletion, rearrangement or substitution of one or more amino acids compared to the amino acid sequence of a reference protein or polypeptide, such as a native or wild-type protein. A variant may have one or more amino acid point substitutions, in which a single amino acid at a position has been changed to another amino acid, one or more insertions and/or deletions, in which one or more amino acids are inserted or deleted, respectively, in the sequence of the reference protein, and/or truncations of the amino acid sequence at either or both the amino or carboxy termini. A variant can have the same or a different biological activity compared to the reference protein, or the unmodified protein.

In some cases, a variant can have, for example, at least 80%, 81%, 82%, 83%, 84%, 85%, 86%, 87%, 88%, 89%, 90%, 91%, 92%, 93%, 94%, 95%, 96%, 97%, 98%, 99%, or 100% overall sequence homology to its counterpart reference protein, wherein the reference protein can be naturally occurring or non-naturally occurring, or a derivative or variant of a naturally occurring protein. In some cases, a variant can have at least about 90% overall sequence homology to the wild-type protein. In some cases, a variant exhibits at least about 95%, at least about 98%, at least about 99%, at least about 99.5%, or at least about 99.9% overall sequence identity.

As used herein, "recombinant" can refer to a biomolecule, e.g., a gene or protein, that (1) has been removed from its naturally occurring environment, (2) is not associated with all or a portion of a polynucleotide in which the gene is found in nature, (3) is operatively linked to a polynucleotide which it is not linked to in nature, or (4) does not occur in nature. The term "recombinant" can be used in reference to cloned DNA isolates, chemically synthesized polynucleotide analogs, or polynucleotide analogs that are biologically synthesized by heterologous systems, as well as proteins and/or mRNAs encoded by such nucleic acids. Thus, for example, a protein synthesized by a microorganism is recombinant, for example, if it is synthesized from an mRNA synthesized from a recombinant gene present in the cell.

"Operatively linked" or "operably linked" or "coupled" can refer to a juxtaposition of genetic elements, wherein the elements are in a relationship permitting them to operate in an expected manner. For instance, a promoter can be operatively linked to a coding region if the promoter helps initiate transcription of the coding sequence. There may be intervening residues between the promoter and coding region so long as this functional relationship is maintained.

The term "expression vector" or "expression construct" or "cassette" or "plasmid" or simply "vector" can include any type of genetic construct, including AAV or rAAV vectors, containing a nucleic acid or polynucleotide coding for a gene product in which part or all of the nucleic acid encoding sequence is capable of being transcribed and is adapted for gene therapy. The transcript can be translated into a protein. In some cases, it may be partially translated or not translated. In certain aspects, expression includes both transcription of a gene and translation of mRNA into a gene product. In other aspects, expression only includes transcription of the nucleic acid encoding genes of interest. An expression vector can also comprise control elements operatively linked to the encoding region to facilitate expression of the protein in target cells. The combination of control elements and a gene or genes to which they are operably linked for expression can sometimes be referred to as an "expression cassette," a large number of which are known and available in the art or can be readily constructed from components that are available in the art.

The term "heterologous" can refer to an entity that is genotypically distinct from that of the rest of the entity to which it is being compared. For example, a polynucleotide introduced by genetic engineering techniques into a plasmid or vector derived from a different species can be a heterologous polynucleotide. A promoter removed from its native coding sequence and operatively linked to a coding sequence with which it is not naturally found linked can be a heterologous promoter.

As used herein, "7m8" refers to the 7-mer amino acid sequence LGETTRP.

"7m8 variant" refers to a rAAV, which can be of any serotype, with the amino acid sequence LGETTRP inserted in the solvent exposed GH loop of the capsid protein.

When 7m8 is inserted in a rAAV2 (also referred to as AAV2.7m8), the 7-mer amino acid sequence LGETTRP is inserted into the GH loop of the AAV2 capsid protein, e.g., between positions 587 and 588 of the AAV2 capsid protein. When 7m8 is inserted in a rAAV1 (also referred to as AAV1.7m8), the 7-mer amino acid sequence LGETTRP is inserted into the GH loop of the AAV1 capsid protein, e.g., between amino acids 590 and 591 of the AAV1 capsid protein. When 7m8 is inserted in a rAAV5 (also referred to as AAV5.7m8), the 7-mer amino acid sequence LGETTRP is inserted into the GH loop of the AAV5 capsid protein, e.g., between amino acids 575 and 576 of the AAV5 capsid protein. When 7m8 is inserted in a rAAV6 (also referred to as AAV6.7m8), the 7-mer amino acid sequence LGETTRP is inserted into the GH loop of the AAV6 capsid protein, e.g., between amino acids 590 and 591 of the AAV6 capsid protein. When 7m8 is inserted in a rAAV7 (also referred to as AAV7.7m8), the 7-mer amino acid sequence LGETTRP is inserted into the GH loop of the AAV7 capsid protein, e.g., between amino acids 589 and 590 of the AAV7 capsid protein. When 7m8 is inserted in a rAAV8 (also referred to as AAV8.7m8), the 7-mer amino acid sequence LGETTRP is inserted into the GH loop of the AAV8 capsid protein, e.g., between amino acids 590 and 591 of the AAV8 capsid protein. When 7m8 is inserted in a rAAV9 (also referred to as AAV9.7m8), the 7-mer amino acid sequence LGETTRP is inserted into the GH loop of the AAV9 capsid protein, e.g., between amino acids 588 and 589 of the AAV9 capsid protein. When 7m8 is inserted in a rAAV10 (also referred to as AAV10.7m8), the 7-mer amino acid sequence LGETTRP is inserted into the GH loop of the AAV10 capsid protein, e.g., between amino acids 589 and 590 of the AAV10 capsid protein.

In some cases, disclosed herein are recombinant adeno-associated virus (rAAV) virions for reducing neovascularization comprising: (a) a variant AAV capsid protein, where the variant AAV capsid protein comprises an amino acid modification in a solvent-exposed region of the capsid protein and shows an increased infectivity of retinal cells relative to a corresponding non-variant AAV capsid protein; and (b) a heterologous nucleic acid comprising a nucleotide sequence encoding a gene product or a therapeutic transgene, and where an administration of an effective amount of the rAAV by intravitreal or subretinal injection in an eye of a primate or human subject results in a reduction in neovascularization in the eye.

Also disclosed herein are recombinant adeno-associated virus (rAAV) virions for reducing neovascularization comprising: (a) a variant AAV capsid protein, where the variant AAV capsid protein comprises an amino acid modification in a solvent-exposed region of the AAV capsid protein, and wherein the variant capsid protein confers an increased ability to cross an internal limiting membrane (ILM) in an eye; and (b) a heterologous nucleic acid comprising a nucleotide sequence encoding a gene product, and where an administration of an effective amount of the rAAV by intravitreal or subretinal injection in an eye of a primate or human subject results in a reduction in neovascularization in the eye.

Also disclosed herein are recombinant adeno-associated virus (rAAV) virions, comprising: (a) a variant AAV capsid protein, wherein the variant AAV capsid protein comprises a peptide insertion of LGETTRP after an amino acid position corresponding to 587 in AAV2, and wherein the variant capsid protein confers an increased ability to deliver a gene product across an internal limiting membrane (ILM) of an eye in primates; and (b) a heterologous nucleic acid comprising a nucleotide sequence encoding the gene product.

Also disclosed herein are gene therapy compositions in a unit dose form for treating an ocular condition or disease, comprising: (a) a recombinant adeno-associated virus (rAAV) virion comprising: (i) a variant AAV capsid protein, wherein the variant AAV capsid protein comprises an amino acid modification in a solvent-exposed region of the capsid protein and shows an increased infectivity of retinal cells relative to a corresponding non-variant AAV capsid protein; and (ii) a heterologous nucleic acid comprising a nucleotide sequence encoding a gene product, wherein the gene product when transduced reduces neovascularization in an eye of a primate or human subject; and (b) a pharmaceutically acceptable excipient; where the rAAV virion is in an amount sufficient to at least partially reduce neovascularization when administered by intravitreal or subretinal injection in the eye of the primate as a unit dose.

Also disclosed herein are methods of treating an ocular condition or disease, comprising administering a unit dose of a rAAV gene therapy described herein to a subject, e.g., a human subj ect.

The term "anti-VEGF agent" includes any therapeutic agent, including proteins, polypeptides, peptides, fusion protein, multimeric proteins, gene products, antibody, human monoclonal antibody, antibody fragment, aptamer, small molecule, kinase inhibitor, receptor or receptor fragment, or nucleic acid molecule, that can reduce, interfere with, disrupt, block and/or inhibit the activity or function of an endogenous VEGF and/or an endogenous VEGF receptor (VEGFR), or the VEGF-VEGFR interaction or pathway in vivo. An anti-VEGF agent can be any one of the known therapeutic agents that can reduce new blood vessel growth or formation and/or oedem, or swelling, when delivered into a cell, tissue, or a subject in vivo, e.g., ranibizumab or bevacizumab. In some cases, an anti-VEGF agent can be naturally occurring, non-naturally occurring, or synthetic. In some cases, an anti-VEGF agent can be derived from a naturally occurring molecule that was subsequently modified or mutated to confer an anti-VEGF activity. In some cases, an anti-VEGF agent is a fusion or chimeric protein. In such proteins, functional domains or polypeptides are artificially fused to a moiety or a polypeptide to make a fusion or chimeric protein that can sequester VEGF in vivo or function as a VEGFR decoy. In some cases, an anti-VEGF agent is a fusion or chimeric protein that blocks endogenous VEGFR from interacting with its ligands.

As used herein, "VEGF" can refer to any isoform of VEGF, unless required otherwise, including, but not limited to, VEGF-A, VEGF-B, VEGF-C, VEGF-D, VEGF-E, VEGF-F, or any combination, or any functional fragment or variant thereof. Unless required otherwise, "VEGF" can refer to any member of the VEGF family, including members: VEGF-A, placenta growth factor (PGF), VEGF-B, VEGF-C, and VEGF-D, or any combination, functional fragment, or variant thereof.

As used herein, "VEGF receptor" or "VEGFR" or "VEGF-R" can be used to refer to any one of the receptors of VEGF, including, but not limited to, VEGFR-1 (or Flt-1), VEGFR-2 (or Flk-1/KDR), and VEGFR-3 (or Flt-4). VEGFR can be a membrane bound or soluble form, or a functional fragment or truncation of a receptor.

Examples of anti-VEGF agent include, but are not limited to, ranibizumab, bevacizumab, or any combination, variant, or functional fragment thereof.

The section headings used herein are for organizational purposes only and are not to be construed as limiting the subject matter described.

### Vectors

Various viral vectors can be used in gene therapy, including adenovirus, adeno-associated virus, retrovirus, and lentivirus.

In some cases, pharmaceutical compositions and methods of the disclosure provide for delivery of a nucleic acid sequence (e.g., cDNA sequence) encoding an anti-VEGF agent, a functional fragment or variant thereof, to retinal cells in a human subject or patient in need thereof (e.g., a patient diagnosed with AMD, RVO, DME). Delivery of the nucleic acid of a therapeutic transgene to a patient using a delivery system, such as rAAV or a viral vector, is also referred to as gene therapy.

In some cases, delivery of anti-VEGF agent nucleic acid sequence can be performed using any suitable "vector" (also referred to as "gene delivery" or "gene transfer vehicle"). Vector (e.g., rAAV), delivery vehicle, gene delivery vehicle or gene transfer vehicle, can encompass any suitable macromolecule or complex of molecules comprising a polynucleotide to be delivered to a target cell, e.g., retinal cells, including photoreceptor, a retinal ganglion cell, a Müller cell, a bipolar cell, an amacrine cell, a horizontal cell, or a retinal pigmented epithelium cell. In some cases, a target cell can be any cell to which the nucleic acid molecule or gene is delivered. The polynucleotide to be delivered can comprise a coding sequence of a therapeutic transgene, such as a sequence that encodes ranibizumab.

The composition and methods of the disclosure provide for any suitable method for delivery of anti-VEGF (e.g., ranibizumab) nucleic acid sequence into an eye or retinal cells of a non-human primate or human subject. In some cases, delivery of the nucleic acid molecule, polynucleotide, or gene therapy is formulated or adapted for intravitreal injection into an eye of a non-human primate or human subject.

In some cases, suitable vectors include, but are not limited to, viral vectors such as adenoviruses, adeno-associated viruses (AAV), and retroviruses, retrovirus, lentivirus, liposomes, lipid-containing complexes, nanoparticles, and other macromolecular complexes capable of delivery of a polynucleotide to retinal cells. In some cases, the viral vector comprises a strong eukaryotic promoter operably linked to the polynucleotide e.g., a cytomegalovirus (CMV) promoter or a constitutive promoter.

In some cases, a vector comprises a recombinant viral vector that incorporates one or more nucleic acid molecules. As described herein, nucleic acids refer to polynucleotides. Nucleic acid and polynucleotide may be used interchangeably. In some cases, nucleic acids comprise DNA or RNA. In some cases, nucleic acids include DNA (e.g., cDNA) or RNA for the expression of an anti-VEGF agent or therapeutic transgene. In some cases, RNA can include a transcript of a gene of interest (e.g., ranibizumab), introns, untranslated regions (UTRs), termination sequences and the like. In other cases, DNA can include, but are not limited to, sequences such as promoter sequences, a gene of interest (e.g. ranibizumab), UTRs, termination sequences, and the like. In some cases, a combination of DNA and RNA can be used.

In some cases, the present disclosure provides a recombinant virus, such as recombinant adeno-associated virus (rAAV) as a vector for delivery and expression of ranibizumab, bevacizumab, sFLT-1, or any functional fragment or variant thereof, in a subject.

In some cases, any suitable viral vectors can be engineered or optimized for use with the compositions and methods of the disclosure. For example, recombinant viral vectors derived from adenovirus (Ad) or adeno-associated virus (AAV) can be altered such that it is replication-defective in human or primate subjects. In some cases, hybrid viral vector systems can be obtained using methods known to one skilled in the art and used to deliver a nucleic acid encoding an anti-VEGF agent to retinal cells. In some cases, a viral delivery system or gene therapy can integrate a nucleic acid sequence comprising an anti-VEGF gene into the target cell genome (e.g., genome of retinal cells) and result in stable gene expression of the gene over time. In some cases, the anti-VEGF gene is not integrated into the target cell genome, and is expressed from a plasmid or vector introduced into the target cells.

In some cases, a suitable viral vector for delivering a nucleic acid sequence of an anti-VEGF to retinal cells is AAV or rAAV, which are small non-enveloped single-stranded DNA viruses. rAAV are non-pathogenic human parvoviruses and can be made to be dependent on helper viruses, including adenovirus, herpes simplex virus, vaccinia virus and CMV, for replication. Exposure to wild-type (wt) AAV is not associated or known to cause any human pathologies and is common in the general population, making AAV or rAAV a suitable delivery system for gene therapy. AAV and rAAV used for gene therapy for delivery of a therapeutic transgene, e.g., ranibizumab, can be of any serotype. In some cases, pharmaceutical compositions and methods of the disclosure provide for use of any suitable AAV serotype, including AAV1, AAV2, AAV2.5, AAV3, AAV4, AAV5, AAV6, AAV7, AAV8, AAV9, AAV10, AAV11, AAV12, rh10, AAV-DJ, and any hybrid or chimeric AAV thereof. In some cases, the serotype used is based on tropism of the virus, or infectivity of a target cell of interest. In some cases, AAV2 or rAAV2 is used to deliver a nucleic acid sequence encoding ranibizumab into an eye or retinal cells of a subject via intravitreal or subretinal injection. In some cases, rAAV2.7m8 is used to deliver the nucleic acid sequence of ranibizumab into the retinal cells of a subject.

In some cases, AAV or rAAV viruses, particles, or virions comprising a variant capsid protein having increased infectivity of target cells, e.g. retinal cells, are used to increase transduction of retinal cells or to increase targeting of gene delivery to retinal cells in a subject. In some cases, the rAAV virion comprises an amino acid modification in a capsid protein GH loop/loop IV of the AAV capsid protein. In some cases, the site of modification is a solvent-accessible portion of the GH loop/loop IV of the AAV capsid protein. Several AAV capsid variants are known, including the 7m8 variant. In some cases, a rAAV virion comprises a variant AAV capsid protein that comprises an insertion of from 5 amino acids to 11 amino acids, e.g., 7 amino acid sequence, in the GH loop of a capsid protein relative to a corresponding parental AAV capsid protein, and wherein the variant capsid protein confers increased infectivity of a retinal cell compared to the infectivity of the retinal cell by an AAV virion comprising the corresponding parental or unmodified AAV capsid protein. In some cases, an insertion of any one of the following amino acid sequences can be inserted in the GH loop of a capsid protein: LGETTRP (7m8), NETITRP, KAGQANN, KDPKTTN, KDTDTTR, RAGGSVG, AVDTTKF, and STGKVPN. In some cases, rAAV.7m8 comprising ranibizumab is used for gene therapy.

In some cases, any one of the following amino acid sequences: NETITRP, KAGQANN, KDPKTTN, KDTDTTR, RAGGSVG, AVDTTKF, and STGKVPN can be inserted at the following positions to generate a rAAV variant for use in gene therapy: between positions 587 and 588 of the AAV2 capsid protein; between amino acids 590 and 591 of the AAV1 capsid protein; between amino acids 575 and 576 of the AAV5 capsid protein; between amino acids 590 and 591 of the AAV6 capsid protein; between amino acids 589 and 590 of the AAV7 capsid protein; between amino acids 590 and 591 of the AAV8 capsid protein; between amino acids 588 and 589 of the AAV9 capsid protein; or between amino acids 589 and 590 of the AAV10 capsid protein.

In some cases, the nucleic acid encoding a gene product such as ranibizumab can be under transcriptional control by a promoter that initiates transcription of the gene. In some cases, the promoter is a "strong" or constitutively active promoter, e.g., CMV promoter. In some cases, the connexin 36 promoter is used to drive expression of a therapeutic transgene, e.g., ranibizumab. In some cases, tissue-specific promoters can be used to effect transcription in specific tissues or cells, such as retinal cells, to reduce potential toxicity or undesirable effects to non-targeted cells. In some cases, a recombinant virus and/or plasmid used to generate a rAAV virus can comprise other transcriptional or regulatory elements, such as poly A (polyadenylation) sequence, untranslated regions (UTRs), 3' UTRs, or termination sequences. In some cases, more than one genes can be expressed from the vector or plasmid using internal ribosome entry site (IRES) or similar elements that allow co-expression of two or more proteins or create multigene, or polycistronic mRNA.

In some cases, the rAAV and/or plasmid used to generate rAAV viruses comprises the following nucleic acid elements: a first ITR sequence; a promoter sequence; an intron sequence; a first UTR sequence; a sequence encoding an anti-VEGF transgene; a second UTR sequence; a polyA sequence; and a second ITR sequence. In some cases, a linker sequence is used between each of these nucleic acid elements. In some cases, the sequence encoding an anti-VEGF transgene comprises a sequence encoding the anti-VEGF transgene fusion protein or a functional fragment thereof.

In some cases, the viral vector of the disclosure is measured as vector genomes. In some cases, a unit dose of recombinant viruses of this disclosure comprise between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹² to 2×10¹², between 2×10¹² to 3×10¹², between 3×10¹² to 4×10¹², between 4×10¹² to 5×10¹², between 5×10¹² to 6×10¹², between 6×10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes. In some cases, the rAAV of this disclosure is about 2.1×10¹² vector genomes. In some cases, the rAAV of this disclosure is between 10¹⁰ to 10¹³, between 10¹⁰ to 10¹¹, between 10¹¹ to 10¹², between 10¹² to 10¹³, between 10¹³ to 10¹⁴, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹⁰, between 7×10¹¹ to 9×10¹¹, between 8×10¹¹ to 10×10¹¹, between 1×10¹² to 3×10¹², between 2×10¹² to 4×10¹², between 3×10¹² to 5×10¹⁰ between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², or between 5×10¹² to 1×10¹³ vector genomes.

In some cases, recombinant viruses of this disclosure are about 1E10, about 1.5E10, about 2E10, about 2.5E10, about 3E10, about 3.5E10, about 4E10, about 4.5E10, about 5E10, about 5.5E10, about 6E10, about 6.5E10, about 7E10, about 7.5E10, about 8E10, about 8.5E10, about 9E10, about 9.5E10, about 10E10, about 1E11, about 1.5E11, about 2E11, about 2.5E11, about 3E11, about 3.5E11, about 4E11, about 4.5E11, about 5E11, about 5.5E11, about 6E11, about 6.5E11, about 7E11, about 7.5E11, about 8E11, about 8.5E11, about 9E11, about 9.5E11, about 10E11, about 1E12, about 1.3E12, about 1.5E12, about 2E12, about 2.1E12, about 2.3E12, about 2.5E12, about 2.7E12, about 2.9E12, about 3E12, about 3.1E12, about 3.3E12, about 3.5E12, about 3.7E12, about 3.9E12, about 4E12, about 4.1E12, about 4.3E12, about 4.5E12, about 4.7E12, about 4.9E12, about 5E12, about 5.1E12, about 5.3E12, about 5.5E12, about 5.7E12, about 5.9E12, about 6E12, about 6.1E12, about 6.3E12, about 6.5E12, about 6.7E12, about 6.9E12, about 7E12, about 7.1E12, about 7.3E12, about 7.5E12, about 7.7E12, about 7.9E12, about 8E12, about 8.1E12, about 8.3E12, about 8.5E12, about 8.7E12, about 8.9E12, about 9E12, about 9.1E12, about 9.3E12, about 9.5E12, about 9.7E12, about 9.9E12, about 10E12, about 10.1E12, about 10.3E12, about 10.5E12, about 10.7E12, about 10.9E12, about 11E12, about 11.5E12, about 12E12, about 12.5E12, about 13E12, about 13.5E12, about 14E12, about 14.5E12, about 15E12, about 15.5E12, about 16E12, about 16.5E12, about 17E12, about 17.5E12, about 18E12, about 18.5E12, about 19E12, about 19.5E12, about 20E12, about 20.5E12, about 30E12, about 30.5E12, about 40E12, about 40.5E12, about 50E12, about 50.5E12, about 60E12, about 60.5E12, about 70E12, about 70.5E12, about 80E12, about 80.5E12, about 90E12, about 95E12, or about 100E12, wherein E is a short-hand for base 10 for exponentiation, and xEy refers to x multiplied by base 10 to the y power/exponent.

In some cases, pharmaceutical compositions disclosed herein comprise recombinant viruses of at least 5E11, at least 5.5E11, at least 6E11, at least 6.5E11, at least 7E11, at least 7.5E11, at least 8E11, at least 8.5E11, at least 9E11, at least 9.5E11, at least 10E11, at least 1E12, at least 1.3E12, at least 1.5E12, at least 2E12, at least 2.1E12, at least 2.3E12, at least 2.5E12, at least 2.7E12, at least 2.9E12, at least 3E12, at least 3.1E12, at least 3.3E12, at least 3.5E12, at least 3.7E12, at least 3.9E12, at least 4E12, at least 4.1E12, at least 4.3E12, at least 4.5E12, at least 4.7E12, at least 4.9E12, at least 5E12, at least 5.1E12, at least 5.3E12, at least 5.5E12, at least 5.7E12, at least 5.9E12, at least 6E12, at least 6.1E12, at least 6.3E12, at least 6.5E12, at least 6.7E12, at least 6.9E12, at least 7E12, at least 7.1E12, at least 7.3E12, at least 7.5E12, at least 7.7E12, at least 7.9E12, at least 8E12, at least 8.1E12, at least 8.3E12, at least 8.5E12, at least 8.7E12, at least 8.9E12, at least 9E12, at least 9.1E12, at least 9.3E12, at least 9.5E12, at least 9.7E12, at least 9.9E12, at least 10E12, at least 10.1E12, at least 10.3E12, at least 10.5E12, at least 10.7E12, at least 10.9E12, at least 11E12, at least 11.5E12, at least 12E12, at least 12.5E12, at least 13E12, at least 13.5E12, at least 14E12, at least 14.5E12, at least 15E12, at least 15.5E12, at least 16E12, at least 16.5E12, at least 17E12, at least 17.5E12, at least 18E12, at least 18.5E12, at least 19E12, at least 19.5E12, at least 20E12, at least 20.5E12, at least 30E12, at least 30.5E12, at least 40E12, at least 40.5E12, at least 50E12, at least 50.5E12, at least 60E12, at least 60.5E12, at least 70E12, at least 70.5E12, at least 80E12, at least 80.5E12, at least 90E12, at least 95E12, or at least 100E12 vector genomes, wherein E is a short-hand for base 10 for exponentiation, and wherein xEy refers to x multiplied by base 10 to the y power/exponent.

In some cases, viral vector of the disclosure is measured using multiplicity of infection (MOI). In some cases, MOI refers to the ratio, or multiple of vector or viral genomes to the cells to which the nucleic acid can be delivered. In some cases, the MOI is 1×10⁶. In some cases, recombinant viruses of the disclosure can be at least 1×10¹, 1×10², 1×10³, 1×10⁴, 1×10⁵, 1×10⁶, 1×10⁷, 1×10⁸, 1x10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some cases, recombinant viruses of this disclosure can be from 1×10⁸ to 1×10¹⁵ MOI. In some cases, recombinant viruses of the disclosure can be at most 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷, and 1×10¹⁸ MOI.

In some cases, the nucleic acid may be delivered without the use of a virus (i.e., with a non-viral vector), and may be measured as the quantity of nucleic acid. Generally, any suitable amount of nucleic acid may be used with the pharmaceutical compositions and methods of this disclosure. In some cases, nucleic acid is at least 1 pg, 10 pg, 100 pg, 1 pg, 10 pg, 100 pg, 200 pg, 300 pg, 400 pg, 500 pg, 600 pg, 700 pg, 800 pg, 900 pg, 1 µg, 10 µg, 100 µg, 200 µg, 300 µg, 400 µg, 500 µg, 600 µg, 700 µg, 800 µg, 900 µg, 1 ng, 10 ng, 100 ng, 200 ng, 300 ng, 400 ng, 500 ng, 600 ng, 700 ng, 800 ng, 900 ng, 1 mg, 10 mg, 100 mg, 200 mg, 300 mg, 400 mg, 500 mg, 600 mg, 700 mg, 800 mg, 900 mg 1 g, 2 g, 3 g, 4 g, or 5 g. In some cases, nucleic acid may be at most about 1 pg, 10 pg, 100 pg, 1 pg, 10 pg, 100 pg, 200 pg, 300 pg, 400 pg, 500 pg, 600 pg, 700 pg, 800 pg, 900 pg, 1 µg, 10 µg, 100 µg, 200 µg, 300 µg, 400 µg, 500 µg, 600 µg, 700 µg, 800 µg, 900 µg, 1 ng, 10 ng, 100 ng, 200 ng, 300 ng, 400 ng, 500 ng, 600 ng, 700 ng, 800 ng, 900 ng, 1 mg, 10 mg, 100 mg, 200 mg, 300 mg, 400 mg, 500 mg, 600 mg, 700 mg, 800 mg, 900 mg, 1 g, 2 g, 3 g, 4 g, or 5 g.

In some cases, a self-complementary vector (sc) can be used. The use of self-complementary AAV vectors may bypass the requirement for viral second-strand DNA synthesis and may lead to greater rate of expression of the transgene protein, as provided by Wu, Hum Gene Ther. 2007, 18(2):171-82.

In some aspects, several AAV vectors may be generated to allow selection of the most optimal serotype and promoter for use with the anti-VEGF transgene.

In some cases, the vector can be a targeted vector, especially a targeted rAAV (e.g., AAV2.7m8) that shows higher infectivity of a specific cell, such as retinal cells, or a photoreceptor, a retinal ganglion cell, a Müller cell, a bipolar cell, an amacrine cell, a horizontal cell, or a retinal pigmented epithelium cell. Viral vectors for use in the disclosure can include those that exhibit low toxicity and/or low immunogenicity in a subject and expresses therapeutically effective quantities of an anti-VEGF transgene in a subject, e.g., human patient.

Disclosed herein are pharmaceutical compositions and methods for delivering a nucleic acid encoding an anti-VEGF agent into a target retinal cell of a subject using the a rAAV comprising a 7m8 variant capsid protein, or rAAV2.7m8, and a nucleic acid sequence that encodes an anti-VEGF transgene in a non-human primate or a human subject. In some instances, the delivery of an anti-VEGF agent via gene therapy can be used to at least partially ameliorate or prevent an ocular disease or condition disclosed herein.

In some cases, the increase in retinal cell infectivity of rAAV variant (e.g., the 7m8 variant) is at least 5%, at least 10%, at least 20%, at least 30%, at least 40%, at least 50%, at least 60%, at least 70%, at least 80%, at least 90%, or at least 100% as compared to an AAV virion comprising the corresponding parental or unmodified AAV capsid protein. In some cases, the increase in infectivity of retinal cells is an increase of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, between 5% to 10% as compared to an AAV virion comprising the corresponding parental or unmodified AAV capsid protein.

In some cases, the increase in retinal cell infectivity of a rAAV variant is at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV virion comprising the corresponding parental or unmodified AAV capsid protein. In some cases, the increase in infectivity is at least 2-fold, at least 3-fold, at least 4-fold, at least 5-fold, at least 6-fold, at least 7-fold, at least 8-fold, at least 9-fold, or at least 10-fold as compared to an AAV virion comprising the corresponding parental AAV capsid protein. In some cases, the increase in infectivity is at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV virion comprising the corresponding parental or unmodified AAV capsid protein.

In some cases, the increase in retinal cell infectivity is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold as compared to an AAV virion comprising the corresponding parental or unmodified AAV capsid protein.

In some cases, the increase in retinal cell infectivity is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold as compared to an AAV virion comprising the corresponding parental or unmodified AAV capsid protein.

In some cases, an amino acid modification of a capsid protein described herein can confer an increase in an ability to cross an internal limiting membrane (ILM) in an eye of a primate or human subject as compared to the ability of an AAV virion comprising the corresponding parental or unmodified AAV capsid protein to cross the ILM in the eye of the subject. In some cases, the increase in the ability to cross the ILM is an increase of at least 5%, at least 10%, at least 20%, at least 30%, at least 40%, at least 50%, at least 60%, at least 70%, at least 80%, at least 90%, or at least 100% as compared to an AAV virion comprising the corresponding parental or unmodified AAV capsid protein. In some cases, the increase in the ability to cross the ILM is an increase of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, or between 5% to 10% as compared to the parental or unmodified AAV capsid protein.

In some cases, the increase in the ability to cross the ILM is at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV virion comprising the corresponding parental AAV capsid protein. In some cases, the increase in the ability to cross the ILM is at least 2-fold, at least 3-fold, at least 4-fold, at least 5-fold, at least 6-fold, at least 7-fold, at least 8-fold, at least 9-fold, or at least 10-fold as compared to an AAV virion comprising the corresponding parental AAV capsid protein. In some cases, the increase in the ability to cross the ILM is at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV virion comprising the corresponding parental or unmodified AAV capsid protein.

In some cases, the increase in the ability to cross the ILM is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold as compared to an AAV virion comprising the corresponding parental or unmodified AAV capsid protein.

In some cases, the increase in the ability to cross the ILM is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold as compared to an AAV virion comprising the corresponding parental or unmodified AAV capsid protein.

In some cases, the vector can be a retroviral vector. Retroviral vectors can include Moloney murine leukemia viruses and HIV-based viruses. In some cases a HIV-based viral vector can be used, wherein the HIV-based viral vector comprises at least two vectors wherein the gag and pol genes are from an HIV genome and the env gene is from another virus. In some cases, DNA viral vectors may be used. These vectors can include pox vectors such as orthopox or avipox vectors, herpesvirus vectors such as a herpes simplex I virus (HSV) vector [Geller, A. I. et al., J. Neurochem, 64: 487 (1995); Lim, F., et al., in DNA Cloning: Mammalian Systems, D. Glover, Ed. (Oxford Univ. Press, Oxford England) (1995); Geller, A. I. et al., Proc Natl. Acad. Sci.: U.S.A.: 90 7603 (1993); Geller, A. I., et al., Proc Natl. Acad. Sci. USA: 87:1149 (1990)], Adenovirus Vectors [LeGal LaSalle et al., Science, 259:988 (1993); Davidson, et al., Nat. Genet. 3: 219 (1993); Yang, et al., J. Virol. 69: 2004 (1995)] and Adeno-associated Virus Vectors [Kaplitt, M. G., et al., Nat. Genet. 8:148 (1994.

In some cases, the vector can be a lentiviral vector. Lentiviral vectors for use in the disclosure may be derived from human and non-human (including SIV) lentiviruses. Examples of lentiviral vectors can include nucleic acid sequences required for vector propagation as well as a tissue-specific promoter operably linked to an anti-VEGF protein gene. Nucleic acid sequences may include the viral LTRs, a primer binding site, a polypurine tract, att sites, and an encapsidation site.

In some cases, the vector can be an alphavirus vector. Alphavirus-based vectors such as those made from semliki forest virus (SFV) and sindbis virus (SIN) may also be used in the disclosure. Use of alphaviruses is described in Lundstrom, K., Intervirology 43:247-257, 2000 and Perri et al., Journal of Virology 74:9802-9807, 2000.

In some cases, the vector can be a pox viral vector. Pox viral vectors may introduce a gene into the cell's cytoplasm. Avipox virus vectors may result in only a short term expression of the gene or nucleic acid. Adenovirus vectors, adeno-associated virus vectors and herpes simplex virus (HSV) vectors may be used with the compositions and methods of the disclosure. The adenovirus vector may result in a shorter term expression (e.g., less than about a month) than adeno-associated virus, in some aspects, and may exhibit much longer expression. The particular vector chosen may depend upon the target cell and the condition being treated.

Disclosed herein are compositions and methods for delivering a nucleic acid encoding a gene product of interest into a target cell of a subject. In some instances, the gene product of interest is delivered to the subject after administration of a vector comprising the gene product. In some instances, the delivery of the gene product can be used to at least partially ameliorate or to treat a disease or condition disclosed herein. In some instances, the composition can be used as a gene therapy or is adapted for gene therapy or delivery of an anti-VEGF in vivo.

In some instances, vector, delivery vehicle, gene delivery vehicle, or gene transfer vehicle refer to any suitable macromolecule or complex of molecules comprising a polynucleotide to be delivered to a target cell, tissue, or a subject. In some cases, a target cell may be any cell to which the nucleic acid or gene is delivered.

In some cases, vectors, e.g., naked DNA or a plasmid, can be delivered into a cell, tissue, or subject using micelles; microemulsions; liposomes; nanospheres; nanoparticles; nanocapsules; solid lipid nanoparticles; dendrimers; polyethylenimine derivative and single-walled carbon nanotubes; and other macromolecular complexes capable of mediating delivery of a polynucleotide to a target cell. In some cases, a vector may be an organic or inorganic molecule. In some cases, a vector is a small molecule (i.e., <5 kD), or a macromolecule (i.e., >5 kD).

In some cases, a vector comprises a recombinant viral vector (e.g., rAAV vector) that incorporates one or more nucleic acids. As described herein, nucleic acids can comprise polynucleotides. In some instances, nucleic acids comprise DNA or RNA. In some cases, nucleic acids include DNA or RNA for the expression of a gene product, or an aptamer. In some cases RNA molecules may include a transcript of a gene of interest, introns, untranslated regions, termination sequences and the like. In other cases, DNA molecules may include sequences such as hybrid promoter gene sequences, strong constitutive promoter sequences, a gene of interest, untranslated regions, termination sequences and the like. In some cases, any combination of DNA and RNA may be used.

In some cases, the present disclosure provides a recombinant virus as a vector to mediate the expression of a gene product, or a gene therapy for delivering a gene product to target cells or a subject in vivo, e.g., an eye or vitreous of an eye. Any suitable recombinant viral vector can be engineered to be optimized for use with the compositions and methods of the disclosure. For example, recombinant viral vectors derived from adenovirus (Ad) or adeno-associated virus (AAV) can be used.

Both human and non-human viral vectors can be used and the recombinant viral vector can be altered such that it is replication-defective in humans or in a subject. In some cases, the vector can be a replication-defective adenovirus or rAAV, comprising a polynucleotide having a promoter operably linked to a therapeutic transgene encoding a gene product or a therapeutic agent, such as an anti-VEGF agent.

In some cases, the vector can be a retroviral vector. Retroviral vectors can include Moloney murine leukemia viruses and HIV-based viruses. In some cases a HIV-based viral vector can be used, wherein the HIV-based viral vector comprises at least two vectors wherein the gag and pol genes are from an HIV genome and the env gene is from another virus. In some cases, DNA viral vectors may be used. These vectors can include pox vectors such as orthopox or avipox vectors, herpesvirus vectors such as a herpes simplex I virus (HSV) vector [Geller, A. I. et al., J. Neurochem, 64: 487 (1995); Lim, F., et al., in DNA Cloning: Mammalian Systems, D. Glover, Ed. (Oxford Univ. Press, Oxford England) (1995); Geller, A. I. et al., Proc Natl. Acad. Sci.: U.S.A.: 90 7603 (1993); Geller, A. I., et al., Proc Natl. Acad. Sci. USA: 87:1149 (1990)], Adenovirus Vectors [LeGal LaSalle et al., Science, 259:988 (1993); Davidson, et al., Nat. Genet. 3: 219 (1993); Yang, et al., J. Virol. 69: 2004 (1995)] and Adeno-associated Virus Vectors [Kaplitt, M. G., et al., Nat. Genet. 8:148 (1994)].

In some cases, the vector can be a lentiviral vector. Lentiviral vectors for use in the disclosure may be derived from human and non-human (including SIV) lentiviruses. Examples of lentiviral vectors can include nucleic acid sequences required for vector propagation as well as a tissue-specific promoter operably linked to an anti-VEGF protein gene. Nucleic acid sequences may include the viral LTRs, a primer binding site, a polypurine tract, att sites, and an encapsidation site.

In some cases, adenovirus vectors, adeno-associated virus vectors, and herpes simplex virus (HSV) vectors can be used with the compositions and methods of the disclosure. The particular vector (e.g., lentivirus, adenovirus, or AAV) used can depend upon the target cell, the size of the therapeutic transgene or agent to be expressed from the vector, and/or the condition being treated.

In some cases, the vector is an adeno-associated virus (AAV) vector or is derived from AAV. AAV are small non-enveloped single-stranded DNA viruses. They are non-pathogenic human parvoviruses and may be dependent on helper viruses, including adenovirus, herpes simplex virus, vaccinia virus and CMV, for replication. Exposure to a wild-type AAV may not be associated or known to cause any human pathologies and is common in the general population, usually occurring in the first decade of life in association with an adenoviral infection.

In some instances, the rAAV can be a native or wild-type AAV of serotype 1, 2, 3, 4, 5, 6, 7, 8, 9, 10, 11, 12, or DJ. In some instances, the rAAV can be a chimeric AAV comprising capsid proteins from at least two serotypes. In some cases, a rAAV virus or virion can comprise a variant AAV capsid protein. In some cases, the variant AAV capsid protein can comprise an amino acid modification selected from the group consisting of a substitution, an insertion, a deletion, and any combination thereof; relative to a corresponding parental AAV capsid protein.

In some cases, the rAAV virion can comprise a deletion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 amino acids in a capsid protein relative to a corresponding parental AAV capsid protein. In some cases, the rAAV virion can comprise a deletion of at least 20, at least 25, at least 30, at least 35, at least 40, at least 45, at least 50, at least 55, at least 60, at least 65, at least 70, at least 75, at least 80, at least 85, at least 90, at least 95 or at least 100 amino acids in a capsid protein. In some cases, the rAAV virion can comprise a deletion of at most about 100 amino acids, at most about 200, at most about 300, or at most about 400 amino acids in a capsid protein. In some cases, the rAAV virion can comprise a deletion of from about 1 to about 100, from about 1 to about 90, from about 1 to about 80, from about 1 to about 70, from about 1 to about 60, from about 1 to about 50, from about 1 to about 40, from about 1 to about 30, from about 1 to about 20, from about 1 to about 15, from about 1 to about 10, or from about 1 to about 5 amino acids in a capsid protein. In some cases, the rAAV virion can comprise a deletion of from about 5 amino acids to about 20 amino acids, from about 5 amino acids to about 19 amino acids, from about 5 amino acids to about 18 amino acids, from about 5 amino acids to about 17 amino acids, from about 5 amino acids to about 16 amino acids, from about 5 amino acids to about 15 amino acids, from about 5 amino acids to about 14 amino acids, from about 5 amino acids to about 13 amino acids, from about 5 amino acids to about 12 amino acids, from about 5 amino acids to about 11 amino acids, from about 5 amino acids to about 10 amino acids, from about 5 amino acids to about 9 amino acids, from about 5 amino acids to about 8 amino acids, from about 5 amino acids to about 7 amino acids, or from about 5 amino acids to about 6 amino acids in a capsid protein.

In some cases, the rAAV virion can comprise an insertion of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 amino acids in a capsid protein relative to a corresponding parental AAV capsid protein. In some cases, the rAAV virion can comprise an insertion of at least 20, at least 25, at least 30, at least 35, at least 40, at least 45, at least 50, at least 55, at least 60, at least 65, at least 70, at least 75, at least 80, at least 85, at least 90, at least 95 or at least 100 amino acids in a capsid protein. In some cases, the rAAV virion can comprise an insertion of at most about 100 amino acids, at most about 200, at most about 300, or at most about 400 amino acids in a capsid protein. In some cases, the rAAV virion can comprise an insertion of from about 1 to about 100, from about 1 to about 90, from about 1 to about 80, from about 1 to about 70, from about 1 to about 60, from about 1 to about 50, from about 1 to about 40, from about 1 to about 30, from about 1 to about 20, from about 1 to about 15, from about 1 to about 10, or from about 1 to about 5 amino acids in a capsid protein. In some cases, the rAAV virion can comprise an insertion of from about 5 amino acids to about 20 amino acids, from about 5 amino acids to about 19 amino acids, from about 5 amino acids to about 18 amino acids, from about 5 amino acids to about 17 amino acids, from about 5 amino acids to about 16 amino acids, from about 5 amino acids to about 15 amino acids, from about 5 amino acids to about 14 amino acids, from about 5 amino acids to about 13 amino acids, from about 5 amino acids to about 12 amino acids, from about 5 amino acids to about 11 amino acids, from about 5 amino acids to about 10 amino acids, from about 5 amino acids to about 9 amino acids, from about 5 amino acids to about 8 amino acids, from about 5 amino acids to about 7 amino acids, or from about 5 amino acids to about 6 amino acids in a capsid protein.

In some cases, the rAAV virion can comprise a substitution of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 amino acids in a capsid protein relative to a corresponding parental AAV capsid protein. In some cases, the rAAV virion can comprise a substitution of from about 1 amino acids to about 20 amino acids, from about 1 amino acids to about 19 amino acids, from about 1 amino acids to about 18 amino acids, from about 1 amino acids to about 17 amino acids, from about 1 amino acids to about 16 amino acids, from about 1 amino acids to about 15 amino acids, from about 1 amino acids to about 14 amino acids, from about 1 amino acids to about 13 amino acids, from about 1 amino acids to about 12 amino acids, from about 1 amino acids to about 11 amino acids, from about 1 amino acids to about 10 amino acids, from about 1 amino acids to about 9 amino acids, from about 1 amino acids to about 8 amino acids, from about 1 amino acids to about 7 amino acids, from about 1 amino acids to about 6 amino acids, from about 1 to about 5 amino acids, from about 1 to about 4 amino acids, from about 1 to about 3 amino acids, or from about 1 to about 2 amino acids in a capsid protein.

In some cases, the rAAV virion can comprise at least about 1, at least about 2, at least about 3, at least about 4, at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, or at least about 20 total amino acid insertions, deletions or substitutions in a capsid protein relative to a corresponding parental, unmodified capsid protein. In some cases, the rAAV virion can comprise at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 total amino acid insertions, deletions or substitutions in a capsid protein relative to a corresponding parental, unmodified capsid protein. In some cases, the rAAV virion can comprise at least about 100, at least about 200, at least about 300, or at least about 400 total amino acid insertions, deletions or substitutions in a capsid protein relative to a corresponding parental, unmodified capsid protein.

In some cases, the rAAV virion comprises a variant capsid protein with an amino acid sequence that is at least about 50%, at least about 55%, at least about 60%, at least about 65%, at least about 70%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% homologous to a capsid protein of a parental, unmodified AAV capsid protein.

In some cases, the modification can be after amino acid 587 of AAV2, or the corresponding residue of a capsid subunit of another AAV serotype. It should be noted that the residue 587 is based on an AAV2 capsid protein. A modification can also be incorporated at a corresponding site in an AAV serotype other than AAV2 (e.g., AAV8, AAV9, etc.). Those skilled in the art would know, based on a comparison of the amino acid sequences of capsid proteins of various AAV serotypes, where a modification site corresponding to amino acid 587 of AAV2 would be in a capsid protein of any given AAV serotype. See, e.g., GenBank Accession No. NP-049542 for AAV1; GenBank Accession No. AAD13756 for AAV5; GenBank Accession No. AAB95459 for AAV6; GenBank Accession No. YP-077178 for AAV7; GenBank Accession No. YP-077180 for AAV8; GenBank Accession No. AAS99264 for AAV9 and GenBank Accession No. AAT46337 for AAV10.

In some cases, the amino acid modification is an insertion of from about 5 amino acids to about 11 amino acids in a protein GH loop or loop IV. In some cases, the amino acid modification is an insertion that comprises one or more amino acids that disrupt a solvent-exposed region of the capsid protein to include a GH loop. In some specific cases, the modification comprises an insertion of amino acid sequence LGETTRP between residue 587 and 588 in VP1 of AAV2. In some cases, other insertions or AAV2 variants can be used as a vector or gene therapy for delivering an anti-VEGF agent into a subject, e.g., sFLT-1, ranibizumab, or bevacizumab.

In some cases, an amino acid modification of a capsid protein described herein can confer an increase in infectivity of an ocular cell compared to the infectivity of the retinal cell by an AAV virion comprising the corresponding parental or unmodified AAV capsid protein. In some cases, the ocular cell can be a photoreceptor cell (e.g., rods; cones). In some cases, the ocular cell can be a retinal ganglion cell (RGC). In some cases, the retinal cell can be a retinal pigment epithelium (RPE) cell. In some cases, the ocular cell can be a Müller cell. In some cases, the ocular cell can be an astrocyte. In some cases, the retinal cells can include amacrine cells, bipolar cells, or horizontal cells.

In some cases, the increase in infectivity is an increase of at least about 5%, at least about 10%, at least about 20%, at least about 30%, at least about 40%, at least about 50%, at least about 60%, at least about 70%, at least about 80%, at least about 90%, or at least about 100% as compared to an AAV virion comprising the corresponding parental AAV capsid protein. In some cases, the increase in infectivity is an increase of between about 5% to about 100%, between about 5% to about 95%, between about 5% to about 90%, between about 5% to about 85%, between about 5% to about 80%, between about 5% to about 75%, between about 5% to about 70%, between about 5% to about 65%, between about 5% to about 60%, between about 5% to about 55%, between about 5% to about 50%, between about 5% to about 45%, between about 5% to about 40%, between about 5% to about 35%, between about 5% to about 30%, between about 5% to about 25%, between about 5% to about 20%, between about 5% to about 15%, or between about 5% to about 10% as compared to an AAV virion comprising the corresponding parental AAV capsid protein.

In some cases, the increase in infectivity is at least about 1-fold, at least about 1.1-fold, at least about 1.2-fold, at least about 1.3-fold, at least about 1.4-fold, at least about 1.5-fold, at least about 1.6-fold, at least about 1.7-fold, at least about 1.8-fold, at least about 1.9-fold, or at least about 2-fold compared to an AAV virion comprising the corresponding parental AAV capsid protein. In some cases, the increase in infectivity is at least about 2-fold, at least about 3-fold, at least about 4-fold, at least about 5-fold, at least about 6-fold, at least about 7-fold, at least about 8-fold, at least about 9-fold, or at least about 10-fold as compared to an AAV virion comprising the corresponding parental AAV capsid protein. In some cases, the increase in infectivity is at least about 15-fold, at least about 20-fold, at least about 25-fold, at least about 30-fold, at least about 35-fold, at least about 40-fold, at least about 45-fold, at least about 50-fold, at least about 55-fold, at least about 60-fold, at least about 65-fold, at least about 70-fold, at least about 75-fold, at least about 80-fold, at least about 85-fold, at least about 90-fold, or at least about 100-fold compared to an AAV virion comprising the corresponding parental AAV capsid protein.

In some cases, the increase in infectivity is between about 10-fold to about 100-fold, between about 10-fold to about 95-fold, between about 10-fold to about 90-fold, between about 10-fold to about 85-fold, between about 10-fold to about 80-fold, between about 10-fold to about 75-fold, between about 10-fold to about 70-fold, between about 10-fold to about 65-fold, between about 10-fold to about 60-fold, between about 10-fold to about 55-fold, between about 10-fold to about 50-fold, between about 10-fold to about 45-fold, between about 10-fold to about 40-fold, between about 10-fold to about 35-fold, between about 10-fold to about 30-fold, between about 10-fold to about 25-fold, between about 10-fold to about 20-fold, or between about 10-fold to about 15-fold as compared to an AAV virion comprising the corresponding parental AAV capsid protein.

In some cases, the increase in infectivity is between about 2-fold to about 20-fold, between about 2-fold to about 19-fold, between about 2-fold to about 18-fold, between about 2-fold to about 17-fold, between about 2-fold to about 16-fold, between about 2-fold to about 15-fold, between about 2-fold to about 14-fold, between about 2-fold to about 13-fold, between about 2-fold to about 12-fold, between about 2-fold to about 11-fold, between about 2-fold to about 10-fold, between about 2-fold to about 9-fold, between about 2-fold to about 8-fold, between about 2-fold to about 7-fold, between about 2-fold to about 6-fold, between about 2-fold to about 5-fold, between about 2-fold to about 4-fold, or between about 2-fold to about 3-fold as compared to an AAV virion comprising the corresponding parental AAV capsid protein.

In some cases, an amino acid modification of a capsid protein described herein can confer an increase in an ability to cross an internal limiting membrane (ILM) in an eye of a subject compared to the ability of an AAV virion comprising the corresponding parental or unmodified AAV capsid protein to cross the ILM in the eye of the subject.

In some cases, the increase in the ability to cross the ILM is an increase of at least about 5%, at least about 10%, at least about 20%, at least about 30%, at least about 40%, at least about 50%, at least about 60%, at least about 70%, at least about 80%, at least about 90%, or at least about 100% as compared to an AAV virion comprising the corresponding parental AAV capsid protein. In some cases, the increase in the ability to cross the ILM is an increase of between about 5% to about 100%, between about 5% to about 95%, between about 5% to about 90%, between about 5% to about 85%, between about 5% to about 80%, between about 5% to about 75%, between about 5% to about 70%, between about 5% to about 65%, between about 5% to about 60%, between about 5% to about 55%, between about 5% to about 50%, between about 5% to about 45%, between about 5% to about 40%, between about 5% to about 35%, between about 5% to about 30%, between about 5% to about 25%, between about 5% to about 20%, between about 5% to about 15%, or between about 5% to about 10% as compared to an AAV virion comprising the corresponding parental AAV capsid protein.

In some cases, the increase in the ability to cross the ILM is at least about 1-fold, at least about 1.1-fold, at least about 1.2-fold, at least about 1.3-fold, at least about 1.4-fold, at least about 1.5-fold, at least about 1.6-fold, at least about 1.7-fold, at least about 1.8-fold, at least about 1.9-fold, or at least about 2-fold compared to an AAV virion comprising the corresponding parental AAV capsid protein. In some cases, the increase in the ability to cross the ILM is at least about 2-fold, at least about 3-fold, at least about 4-fold, at least about 5-fold, at least about 6-fold, at least about 7-fold, at least about 8-fold, at least about 9-fold, or at least about 10-fold as compared to an AAV virion comprising the corresponding parental AAV capsid protein. In some cases, the increase in the ability to cross the ILM is at least about 15-fold, at least about 20-fold, at least about 25-fold, at least about 30-fold, at least about 35-fold, at least about 40-fold, at least about 45-fold, at least about 50-fold, at least about 55-fold, at least about 60-fold, at least about 65-fold, at least about 70-fold, at least about 75-fold, at least about 80-fold, at least about 85-fold, at least about 90-fold, or at least about 100-fold compared to an AAV virion comprising the corresponding parental AAV capsid protein.

In some cases, the increase in the ability to cross the ILM is between about 10-fold to about 100-fold, between about 10-fold to about 95-fold, between about 10-fold to about 90-fold, between about 10-fold to about 85-fold, between about 10-fold to about 80-fold, between about 10-fold to about 75-fold, between about 10-fold to about 70-fold, between about 10-fold to about 65-fold, between about 10-fold to about 60-fold, between about 10-fold to about 55-fold, between about 10-fold to about 50-fold, between about 10-fold to about 45-fold, between about 10-fold to about 40-fold, between about 10-fold to about 35-fold, between about 10-fold to about 30-fold, between about 10-fold to about 25-fold, between about 10-fold to about 20-fold, or between about 10-fold to about 15-fold as compared to an AAV virion comprising the corresponding parental AAV capsid protein.

In some cases, the increase in the ability to cross the ILM is between about 2-fold to about 20-fold, between about 2-fold to about 19-fold, between about 2-fold to about 18-fold, between about 2-fold to about 17-fold, between about 2-fold to about 16-fold, between about 2-fold to about 15-fold, between about 2-fold to about 14-fold, between about 2-fold to about 13-fold, between about 2-fold to about 12-fold, between about 2-fold to about 11-fold, between about 2-fold to about 10-fold, between about 2-fold to about 9-fold, between about 2-fold to about 8-fold, between about 2-fold to about 7-fold, between about 2-fold to about 6-fold, between about 2-fold to about 5-fold, between about 2-fold to about 4-fold, or between about 2-fold to about 3-fold as compared to an AAV virion comprising the corresponding parental AAV capsid protein.

One advantage of gene therapy is that it requires less frequent administration of a therapeutic agent such as an anti-VEGF agent as disclosed herein, and provides for a prolonged or continued release of the therapeutic agent as compared to conventional methods that administer proteins. Gene therapy that utilizes vectors, e.g., AAV2.7m8, that target a specific tissue or cell type of interest can also minimize off-target effects, or provide a more targeted delivery of a therapeutic agent such as an anti-VEGF agent. With prolonged or sustained delivery of anti-VEGF agent in vivo via gene therapy, one would be able to administer the pharmaceutical composition not more than once in at least 2, 3, 4, 5, 6, 7, 8, 9, 10 or more years.

### Therapeutic Agents

In some cases, a gene therapy is used to deliver a therapeutic transgene having an anti-VEGF activity that is suitable for or adapted for administration to an eye or vitreous of an eye of a non-human primate or a human subject. In some cases, rAAV comprising a capsid variant (e.g., AAV2.7m8) described herein comprises a heterologous nucleic acid sequence that encodes an anti-VEGF agent is used to deliver the sequence of the anti-VEGF gene into retinal cells upon intravitreal or subretinal injection to a subject. In some cases, the rAAV comprising the anti-VEGF gene is formulated for gene therapy and intravitreal injection. In some cases, the anti-VEGF gene refers to a functional fragment or a variant thereof. In some cases, the nucleic acid sequence of anti-VEGF agents, such as sFLT-1, ranibizumab, or bevacizumab, is derived from its amino acid sequence, which is readily available. In some cases, the nucleic acid sequence of anti-VEGF agents, such as sFLT-1, ranibizumab, or bevacizumab, is further codon optimized to improve its expression in a subject. In some cases, the nucleic acid sequence and/or the amino acid sequence of an anti-VEGF agent is modified to enhance its activity, expression, stability, and/or solubility in vivo.

Codon optimization can be achieved with any method known in the art. Codon optimization refers to a process of modifying a nucleic acid sequence for enhanced expression of a gene in target or host cells of interest, e.g., human retinal cells, by replacing at least one codon (e.g., about or more than 1, 2, 3, 4, 5, 10, 15, 20, 25, 50, 100 or more codons) of a native sequence with codons that are used more frequently or are most frequently used in the host cell while maintaining the native amino acid sequence. Codon usage tables are readily available, including for examples, GenScript Codon Usage Frequence Table Tool at http://www.genscript.com/tools/codon-frequency-table; Codon Usage Database at http://www.kazusa.or.jp/codon/; and Nakamura, Y., et al. "Codon usage tabulated from the international DNA sequence databases: status for the year 2000" Nucl. Acids Res. 28:292 (2000).

In some cases, the amino acid sequence of an anti-VEGF agent encoded in a gene therapy is at least 75%, 80%, 85%, 90%, 91%, 92%, 93%, 94%, 95%, 96%, 97%, 98%, 99%, 99.9%, 99.99% or 100% homologous to amino acid sequence of any one of the following anti-VEGF agents: sFLT-1, ranibizumab, or bevacizumab. In some cases, the nucleic acid sequence used in a gene therapy or rAAV disclosed herein is compared to the corresponding cDNA sequence of the amino acid sequence of any one of sFLT-1, ranibizumab, or bevacizumab, and shows at least 75%, 80%, 85%, 90%, 91%, 92%, 93%, 94%, 95%, 96%, 97%, 98%, 99%, 99.9%, 99.99% or 100% sequence homology between the nucleic acid sequences of any one of sFLT-1, ranibizumab, or bevacizumab. In some cases, an anti-VEGF expressed from the gene therapy is at least 80%, 85%, 90%, 91%, 92%, 93%, 94%, 95%, 96%, 97%, 98%, 99%, 99.9%, 99.99% or 100% spatially homologous to any one of sFLT-1, ranibizumab, or bevacizumab (e.g., in terms of its secondary, tertiary, and quaternary structure or conformation). In some cases, anti-VEGF agent of the pharmaceutical compositions and methods disclosed herein is at most 80%, 85%, 90%, 91%, 92%, 93%, 94%, 95%, 96%, 97%, 98%, 99%, 99.9%, 99.99% or 100% spatially homologous to any one of sFLT-1, ranibizumab, or bevacizumab used in the standard of care (e.g., secondary, tertiary, and quaternary structure or conformation).

In some instances, anti-VEGF agent as included in a gene therapy based on a rAAV comprises a capsid variant as disclosed herein (e.g., the 7m8 variant), encodes a protein, fusion protein, or polypeptide that has at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 100% homology to the corresponding cDNA sequences of the anti-VEGF agent (e.g., sFLT-1, ranibizumab, or bevacizumab). In some cases, methods and pharmaceutical compositions disclosed herein comprise sFLT-1, ranibizumab, or bevacizumab, or a functional fragment or variant or mutant thereof. In some cases, the nucleic acid sequence of any of sFLT-1, ranibizumab, or bevacizumab is modified or codon-optimized to enhance its activity, expression, and/or solubility in vivo.

In some cases, AAV2.7m8 is used as a gene therapy or a delivery system for any one of sFLT-1, ranibizumab, or bevacizumab. AAV2.7m8-sVEGFR-1 refers to rAAV2 comprising the 7m8 insertion between positions 587 and 588 of capsid protein VP1 of AAV2 and a nucleic acid sequence encoding sVEGFR-1. AAV2.7m8- ranibizumab refers to rAAV2 comprising the 7m8 insertion between positions 587 and 588 of capsid protein VP1 of AAV2 and a nucleic acid sequence encoding ranibizumab.

The present disclosure contemplates methods and pharmaceutical compositions as disclosed herein comprising one or more therapeutic agents. In some cases, the therapeutic agent is an anti-VEGF agent. In some cases, the anti-VEGF agent is expressed from a rAAV vector or gene therapy, or is delivered into a target cell, tissue, or a subject in vivo. Gene therapy has the advantage of providing the therapeutic agent, e.g., anti-VEGF agent, for a prolonged period of time in vivo, which decreases the need for repeated injections as compared to administration of a protein-based therapy. Such advantage of gene therapy can lead to a more sustained delivery of the therapeutic agent in vivo, which provides an improvement over the current standard of care. Additionally, a gene therapy can also provide a more targeted delivery of the therapeutic agent in vivo, e.g., to target cells, and minimize off-target effects.

In some cases, a gene product disclosed herein can be a polypeptide that when expressed can result in a reduction in neovascularization in an eye of a subject. In some cases, the expressed polypeptide can be an anti-vascular endothelial growth factor (VEGF) protein or peptide, or an angiogenesis inhibitor.

In some cases, a gene product disclosed herein can be an anti-VEGF antibody or a fragment thereof that can target or at least partially inhibit VEGF. In some instances, the antibody can be a full length antibody, comprising both a variable region and Fc region. In some instances, the antibody can be a single chain fv fragment. In some instances, the antibody can have a defined binding affinity to a VEGF epitope. In some instances, the antibody can have a Kd of at least about 1 mM, at least about 100 µM, at least about 10 µM, at least about 1 µM, at least about 100 nM, at least about 10 nM, at least about 1 nM, at least about 100 pM, at least about 10 pM, or at least about 1 pM. In some instances, an anti-VEGF agent binds to an endogenous VEGF or VEGFR stronger than the corresponding endogenous VEGFR or VEGF. Stronger binding of the anti-VEGF agent allows the anti-VEGF agent to sequester endogenous VEGF or to block endogenous proteins from interacting with an endogenous VEGFR.

In some cases, the anti-VEGF antibody can be a humanized monoclonal antibody. In some instances, the humanized monoclonal antibody can be rhuMab. In some cases, the anti-VEGF antibody can be ranibizumab, a monoclonal antibody fragment, or a variant or fragment thereof. In some cases, the anti-VEGF antibody can be bevacizumab, a recombinant humanized monoclonal antibody, or a variant or fragment thereof. In some cases, the anti-VEGF agent is PAN-90806, or a variant or fragment thereof. In some cases, the therapeutic agent is a nucleic acid sequence that encodes one or more polypeptides comprising an anti-VEGF agent as disclosed herein, e.g., an antibody, antibody fragment, monoclonal antibody, a humanized monoclonal antibody, fusion protein, aptamer, etc. In some cases, the anti-VEGF agent is a soluble receptor decoy that binds to VEGF, or a soluble form of one more VEGF receptors that can sequester VEGF in vivo.

In some cases, a therapeutic agent disclosed herein can be a nucleic acid such as an aptamer, an interfering RNA, an mRNA, and the like. In some cases, the aptamer can be pegaptanib. In some cases, the therapeutic agent can be a steroid or a small molecule. In some instances, the steroid can be a corticosteroid. Examples of corticosteroids can include triamcinolone, dexamethasone, fluocinolone acetonide, cortisone, prednisolone, flumetholone, and derivatives thereof. In some instances, the steroid can be an anti-inflammatory steroid.

The recombinant virus, gene therapy, pharmaceutical compositions, and methods of the present disclosure can comprise the sequence encoding an anti-VEGF protein, including, but not limited to, the VEGF-binding proteins or functional fragments thereof as disclosed in U.S. Pat. Nos. 5,712,380, 5,861,484 and 7,071,159, and also as described in U.S. Pub. No. 2014/0371438. In some cases, an anti-VEGF protein includes the sFLT-1 protein, ranibizumab, or bevacizumab as described herein.

In some cases, the gene therapy, pharmaceutical compositions, and methods of the present disclosure can comprise the sequence encoding an anti-VEGF protein, e.g., sFlt-1. In some cases, an anti-VEGF agent includes, but is not limited to, functional fragments of sFlt-1, including sequences of sFlt-1 domain 2 or the sequence. An anti-VEGF agent can include sequences or polypeptides expressed from DNA encoding such sequences using the genetic code, a standard technique that is understood by those skilled in the art.

As used herein, "sFlt-1 protein" or "sFlt" or "sVEGFR-1" are used interchangeably to refer to a polypeptide sequence, or a functional fragment or variant thereof, with at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 100% homology to the human sFLT-1 sequence, such that the sFlt-1 protein or polypeptide binds to VEGF and/or the VEGF receptor in vivo. **FIG. 2** illustrates a nucleic acid sequence of sFlt-1. Homology refers to the % conservation of residues of an alignment between two sequences (e.g. naturally occurring human sFLT-1 protein may include any suitable variants of sFLT-1, including, but not limited to functional fragments, sequences comprising insertions, deletions, substitutions, pseudofragments, pseudogenes, splice variants or artificially optimized sequences. In some cases, "sFLT-1 protein" is at least about 90%, 91%, 92%, 93%, 94%, 95%, 96%, 97%, 98%, 99%, or 100% homologous to the naturally occurring human sFLT-1 protein sequence. In some cases, "sFLT-1 protein" is at most about 90%, 91%, 92%, 93%, 94%, 95%, 96%, 97%, 98%, 99%, or 100% homologous to the naturally occurring human sFLT-1 protein sequence. In some cases, "sFLT-1 protein" is at least about 90%, 91%, 92%, 93%, 94%, 95%, 96%, 97%, 98%, 99%, or 100% spatially homologous to the naturally occurring human sFLT-1 protein conformation. In some cases, "sFLT-1 protein" is at most about 90%, 91%, 92%, 93%, 94%, 95%, 96%, 97%, 98%, 99%, or 100% spatially homologous to the naturally occurring human sFLT-1 protein conformation.

In some cases, the soluble truncated form of the VEGF receptor FLT-1, sFLT-1, is the only known endogenous inhibitor of VEGF. sFLT-1 can be generated by alternative RNA splicing and lacks the membrane-proximal immunoglobulin-like domain, the transmembrane spanning region and the intracellular tyrosine-kinase domain.

In some cases, administration of a gene therapy or pharmaceutical composition as disclosed herein comprising sFLT-1 can inhibit or reduce VEGF or its activity in vivo by binding or sequestering endogenous VEGF, or by forming inactive heterodimers with membrane-spanning isoforms of the VEGF receptors FLTt-1 and FLK-1/KDR. These properties of sFLT-1 have been described in Kendall and Thomas, 1993; Proc Natl Acad. Sci. 90: 10705-10709. In some cases, functional fragments of sFLT-1 can be used instead of the full-length protein. In some cases, the VEGF binding domain (domain 2), or alternatively KDR, or another family member, can be used to bind and inactivate VEGF.

In some cases, the methods and pharmaceutical compositions of the present disclosure comprise an anti-VEGF agent that is bevacizumab or ranibizumab, a functional fragment or variant thereof. Catt Research, Group; Martin, DF; Maguire, MG; Ying, GS; Grunwald, JE; Fine, SL; Jaffe, GJ (2011). "Ranibizumab and Bevacizumab for Neovascular Age-Related Macular Degeneration". New England Journal of Medicine. 364 (20): 1897-1908.

Ranibizumab light chain and heavy chain amino acid sequences are publicly available at DrugBank database, accession number DB01270:
>Ranibizumab Light Chain 
DIQLTQSPSSLSASVGDRVTITCSASQDISNYLNWYQQKPGKAPKVLIYFTSSLHSGVPSR
FSGSGSGTDFTLTISSLQPEDFATYYCQQYSTVPWTFGQGTKVEIKRTVAAPSVFIFPPSD
 
EQLKSGTASVVCLLNNFYPREAKVQWKVDNALQSGNSQESVTEQDSKDSTYSLSSTLTL
SKADYEKHKVYACEVTHQGLSSPVTKSFNRGEC
>Ranibizumab Heavy Chain

EVQLVESGGGLVQPGGSLRLSCAASGYDFTHYGMNWVRQAPGKGLEWVGWINTYTGE
PTYAADFKRRFTFSLDTSKSTAYLQMNSLRAEDTAVYYCAKYPYYYGTSHWYFDVWG
QGTLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHL

Bevacizumab light chain and heavy chain amino acid sequences are publicly available at DrugBank database, access number DB00112 (BTD00087, BIOD00087):
>Bevacizumab Light Chain 
DIQMTQSPSSLSASVGDRVTITCSASQDISNYLNWYQQKPGKAPKVLIYFTSSLHSGVPSR
FSGSGSGTDFTLTISSLQPEDFATYYCQQYSTVPWTFGQGTKVEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC
>Bevacizumab Heavy Chain

EVQLVESGGGLVQPGGSLRLSCAASGYTFTNYGMNWVRQAPGKGLEWVGWINTYTGE
PTYAADFKRRFTFSLDTSKSTAYLQMNSLRAEDTAVYYCAKYPHYYGSSHWYFDVWG
QGTLVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

Bevacizumab is a recombinant humanized monoclonal IgG1 antibody that binds to all VEGF-A isoforms and blocks angiogenesis by inhibiting VEGF-A. Los, M.; Roodhart, J. M. L.; Voest, E. E. (2007). "Target Practice: Lessons from Phase III Trials with Bevacizumab and Vatalanib in the Treatment of Advanced Colorectal Cancer". The Oncologist. 12 (4): 443-50; Shih, T; Lindley, C (November 2006). "Bevacizumab: an angiogenesis inhibitor for the treatment of solid malignancies." Clinical therapeutics. 28 (11): 1779-802.

Ranibizumab is a recombinant humanized IgG1 kappa isotype monoclonal antibody fragment and binds to all VEGF-A isoforms with a higher affinity than bevacizumab. Ranibizumab lacks an Fc region. In some cases, an anti-VEGF agent is bevacizumab or ranibizumab, or a functional fragment or variant thereof. In some cases, an anti-VEGF agent is at least about 80%, 85%, 90%, 91%, 92%, 93%, 94%, 95%, 96%, 97%, 98%, 99%, 99.9%, 99.99% or 100% homologous to bevacizumab or ranibizumab in amino acid and/or nucleic acid (e.g., cDNA) sequence. In some cases, an anti-VEGF agent is at most about 90%, 91%, 92%, 93%, 94%, 95%, 96%, 97%, 98%, 99%, 99.9%, 99.99% or 100% homologous to bevacizumab or ranibizumab in amino acid and/or nucleic acid (e.g., cDNA) sequences known in the field. In some cases, an anti-VEGF agent is at least about 90%, 91%, 92%, 93%, 94%, 95%, 96%, 97%, 98%, 99%, 99.9%, 99.99% or 100% spatially homologous to bevacizumab or ranibizumab protein conformation, including secondary, tertiary, or quaternary structures.

Given an amino acid sequence, one can readily generate the corresponding cDNA or nucleic acid sequence to use in a gene therapy disclosed herein. Methods for reverse translating an amino acid sequence include EMBOSS Protein Sequence Back-translation tool available at http://www.ebi.ac.uk/Tools/st/. In some cases, a nucleic acid sequence of an anti-VEGF agent is codon-optimized using any of the techniques known it the art, for example, GenScript Codon Usage Frequence Table Tool at http://www.genscript.com/tools/codon-frequency-table; Codon Usage Database at http://www.kazusa.or.jp/codon/; and Nakamura, Y., et al. "Codon usage tabulated from the international DNA sequence databases: status for the year 2000" Nucl. Acids Res. 28:292 (2000).

### Pharmaceutical Compositions

In some cases, a pharmaceutical composition is a formulation containing one or more active ingredients, e.g., AAV2.7m8 comprising a nucleic acid sequence that encodes the an anti-VEGF agent, or a fragment or variant thereof, as well as one or more excipients, carriers, stabilizers, or bulking agents, which are suitable for administration to a human patient via intravitreal or subretinal injection to achieve a desired therapeutic or prophylactic effect.

In some cases, the pharmaceutical compositions comprising rAAV, or AAV2.7m8 and a nucleic acid sequence that encodes an anti-VEGF agent, are supplied as a reconstituted solution or suspension. In other cases, the pharmaceutical compositions comprising rAAV, or AAV2.7m8 and a nucleic acid sequence that encodes an anti-VEGF agent, are supplied in a lyophilized form, and is reconstituted before administration to a patient. In some cases, method of treatment or prevention of an eye disease or condition as disclosed herein comprises first reconstituting, dissolving, or solubilizing a lyophilized pharmaceutical composition comprising rAAV (e.g., AAV2.7m8) and a nucleic acid sequence that encodes an anti-VEGF agent in a buffer. In some cases, such lyophilized pharmaceutical composition comprising rAAV (e.g., AAV2.7m8) and a nucleic acid sequence that encodes an anti-VEGF agent as disclosed herein, can further comprise a cryoprotectant, surfactant, salt, a stabilizer, or any combination thereof.

In some cases, the pharmaceutical compositions comprising rAAV, or AAV2.7m8 and a nucleic acid sequence that encodes an anti-VEGF agent is supplied as a suspension. In some cases, the suspension is refrigerated. In some cases, the suspension is a solution. In some cases, a homogenous solution containing the pharmaceutical composition is supplied as a pre-filled syringe. In some cases, pharmaceutical compositions disclosed herein are supplied as a suspension. In some cases, the suspension is refrigerated. In some cases, method of treatment or prevention of an eye disease or condition as disclosed herein comprises warming the refrigerated suspension to room temperature and/or agitating the suspension to ensure even distribution before administering or intravitreal injection to a patient. In some cases, the suspension is diluted before administering to a patient. In some cases, such pharmaceutical composition comprises a surfactant, salt, a stabilizer, or any combination thereof. In some cases, a suspension containing the pharmaceutical composition is supplied as a pre-filled syringe.

In some cases, the gene therapy or pharmaceutical compositions described herein is provided as a suspension or as a refrigerated suspension. In some cases, the suspension comprises a pharmaceutically acceptable excipient, e.g., surfactant, glycerol, non-ionic surfactant, buffer, glycol, salt, and any combination thereof. In some cases, hydrochloric acid and sodium hydroxide are used to adjust the pH of the solution. In some cases, the refrigerated suspension is at a neutral pH, or at a pH between 6.5 to 7.5. In some cases, the pH of the refrigerated suspension is slightly basic (e.g., pH about 7.5, 8, 8.2, 8.4, 8.5, or 9). In some cases, the pH of the refrigerated suspension or solution is slightly acidic (e.g., pH about 6.5, 6.3, 6.1, 6, 5.5, or 5). In some cases, the refrigerated suspension is a solution. In some cases, the refrigerated suspension comprises micelles. In some cases, refrigerated suspension is agitated before administration.

In some cases, a gene therapy comprising rAAV (e.g., AAV2.7m8) and an anti-VEGF agent as disclosed herein is supplied as a kit, comprising lyophilized or freeze-dried pharmaceutical composition disclosed herein and a buffered solution for dissolving, diluting or reconstituting the lyophilized pharmaceutical composition. In some cases, a kit comprises freeze-dried or lyophilized pharmaceutical composition comprising rAAV (e.g., AAV2.7m8) and a solution for reconstituting the pharmaceutical composition to a desired concentration or volume. In some cases, the kit includes a buffer that helps to prevent aggregation upon reconstituting the pharmaceutical composition disclosed herein. In some cases, the gene therapy comprising the anti-VEGF agent is provided as a suspension. In some cases, the pharmaceutical composition is provided in a pre-filled syringe. In some cases, a kit comprises a dual-chamber syringe wherein one of the chambers contains a buffer for dissolving or diluting the pharmaceutical composition.

In some cases, the kit comprises a syringe for injection. In some cases, the reconstituted solution is filtered before administration. In some cases, the kit comprises a filter or a filter syringe for filtering the pharmaceutical composition in the kit before administration to a patient.

In some cases, for storage stability and convenience of handling, a pharmaceutical composition, comprising rAAV (e.g., AAV2.7m8) and a nucleic acid sequence that encodes an anti-VEGF agent as disclosed herein, can be formulated as a lyophilized (i.e. freeze dried) or vacuum dried powder that can be reconstituted with saline, buffer, or water prior to administration to a subject. Alternately, the pharmaceutical composition can be formulated as an aqueous solution or suspension. A pharmaceutical composition can contain rAAV virions or particles comprising a nucleic acid sequence that encodes an anti-VEGF agent as disclosed herein. In some cases, a different virus or delivery system, e.g., nanoparticles or lipid-based complexes, can be used to deliver the nucleic acid sequence that encodes an anti-VEGF agent as disclosed herein. Various excipients, such as phosphate, PBS, or Tris buffer, glycol, glycerol, saline, surfactant (e.g., pluronic or polysorbate), or any combination thereof, can be used to stabilize a pharmaceutical composition. Additionally, cryoprotectants, such as alcohols, DMSO, glycerol, and PEG can be used as a stabilizer under the freezing or drying conditions of lyophilization, or be used as a stabilizer for making a refrigerated suspension.

In some cases, the lyophilized or a suspension of the pharmaceutical composition comprising an anti-VEGF gene therapy as disclosed herein has a volume (or reconstituted volume) of about 10, 20, 30, 40, 50, 60, 70, 80, 90, 100, 200, 300, 400, 500, 600, 700, 800, 900, or 1000 µL. In some cases, the lyophilized or suspension form of the pharmaceutical composition comprising the an anti-VEGF gene therapy as disclosed herein has a volume of between 0.1 to 0.5 mL, between 0.1 to 0.2 mL, between 0.3 to 0.5 mL, between 0.5-1.0 mL, between 0.5-0.7 mL, between 0.6 to 0.8 mL, between 0.8 to 1 mL, between 0.9 to 1.1 mL, between 1.0 to 1.2, or between 1.0 to 1.5 mL mL. In other cases, the reconstituted volume is no more than about 0.1, 0.2, 0.3, 0.4, 0.5, 0.6, 0.7, 0.8, 0.9, 1.0, 1.1, 1.2, 1.3, 1.4, or 1.5 mL.

In some cases, pharmaceutical compositions disclosed herein are designed, engineered, or adapted for administration to a primate (e.g., non-human primate and human subjects) via intravitreal or subretinal injection. In some cases, a pharmaceutical composition comprising rAAV virions comprising a nucleic acid sequence that encodes an anti-VEGF agent is formulated for intravitreal injection into an eye of a subject. In some cases, the pharmaceutical composition is formulated to a concentration that allows intravitreal injection of a volume not more than about 2, 2.5, 5, 10, 15, 20, 25, 30, 35, 40, 45, 50, 55, 60, 65, 70, 75, 80, 85, 90, 95, 100, 110, 120, 130, 140, 150, 160, 170, 180, 190, or 200 µL. In some cases, methods of treatment disclosed herein comprises intravitreal injection of a volume of about 2, 5, 10, 15, 20, 25, 30, 35, 40, 45, 50, 55, 60, 65, 70, 75, 80, 85, 90, 95, 100, 110, 120, 130, 140, 150 µL of a solution or suspension comprising a rAAV (e.g., AAV2.7m8) comprising a nucleic acid sequence that encodes an anti-VEGF agent as disclosed herein.

In some instances, a AAV2.7m8 virion comprising a nucleic acid sequence of the anti-VEGF transgene described herein can be a component of a gene therapy pharmaceutical composition. In some cases, a rAAV virion of any serotype comprising the 7m8 variant capsid protein as described herein can be used to make a lyophilized pharmaceutical composition or a suspension of the pharmaceutical composition. In some cases, the rAAV virion is rAAV2. In some cases, the lyophilized form or a suspension form of the pharmaceutical composition comprises rAAV2 having a 7m8 variant capsid protein and a DNA sequence that encodes an anti-VEGF agent as disclosed herein.

In some cases, a pharmaceutical composition disclosed herein is adapted for gene therapy or for intravitreal delivery of an anti-VEGF agent as the therapeutic agent in human patients or non-human primates. In some cases, a unit dose of the pharmaceutical composition comprises between 1×10¹⁰ to 1×10¹³ viral genomes (vg). In some cases, a unit dose comprises about 2.1×10¹¹, about 2.1×10¹², or about 2.1×10¹³ vector genome. In some cases, the unit dose of the pharmaceutical composition of the disclosure is 1×10¹⁰ to 3×10¹² vector genomes. In some cases, the unit dose of the pharmaceutical composition of the disclosure is 1×10⁹ to 3×10¹³ vector genomes. In some cases, the unit dose of the pharmaceutical composition of the disclosure is 1× 10¹⁰ to 1× 10¹¹ vector genomes. In some cases, the unit dose of the pharmaceutical composition of the disclosure is 1×10⁸ to 3×10¹⁴ vector genomes. In some cases, the unit dose of the pharmaceutical composition of the disclosure is at least 1×10¹, 1×10², 1×10³, 1×10⁴, 1×10⁵, 1×10⁶, 1×10⁷, 1×10⁸ , 1×10⁹, 1×10¹⁰, 1×10¹¹, 1×10¹², 1×10¹³, 1×10¹⁴, 1×10¹⁵, 1×10¹⁶, 1×10¹⁷, or 1×10¹⁸ vector genomes. In some cases, the unit dose of the pharmaceutical composition of the disclosure is 1×10¹⁰ to 5×10¹³ vector genomes. In some cases, the unit dose of the pharmaceutical composition of the disclosure is at most about 1×10⁸, 1×10⁹, 1×10¹⁰, 1×10¹¹, 1×10¹², 1×10¹³, 1×10¹⁴, 1×10¹⁵, 1×10¹⁶, 1×10¹⁷, and 1×10¹⁸ vector genomes.

In some cases, a unit dose of the pharmaceutical composition of the disclosure can be measured as pfu (plaque forming units). In some cases, the pfu of the unit dose of the pharmaceutical composition of the disclosure can be about 1×10⁸ to about 1×10¹² pfu. In some cases, the pfu of the unit dose of the pharmaceutical composition of the disclosure can be at least about 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, 9×10¹⁰, 1×10¹¹, 2×10¹¹, 3×10¹¹, 4×10¹¹, 5×10¹¹, 6×10¹¹, 7×10¹¹, 8×10¹¹, 9×10¹¹ or 1×10¹² pfu. In some cases, the pfu of the unit dose of the pharmaceutical composition of the disclosure can be at most about 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, 9×10¹⁰, 1×10¹¹, 2×10¹¹, 3×10¹¹, 4×10¹¹, 5×10¹¹, 6×10¹¹, 7×10¹¹, 8×10¹¹, 9×10¹¹ or 1×10¹² pfu.

In some cases, the viral vector of the disclosure may be measured as vector genomes (vg). In some cases, the unit dose of the pharmaceutical composition of the disclosure can be 1×10¹⁰ to 1×10¹³ vector genomes. In some cases, the unit dose of the pharmaceutical composition of the disclosure can be 1×10⁹ to 1×10¹⁴ vector genomes. In some cases, the unit dose of the pharmaceutical composition of the disclosure can be 1×10¹⁰, to 1×10¹¹ vector genomes. In some cases, the unit dose of the pharmaceutical composition of the disclosure can be 1×10⁸ to 1×10¹⁵ vector genomes. In some cases, the unit dose of the pharmaceutical composition of the disclosure is at least 1×10¹, 1×10², 1×10³, 1×10⁴, 1×10⁵, 1×10⁶, 1×10⁷, 1×10⁸, 1x10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ vector genomes. In some cases, the unit dose of the pharmaceutical composition of the disclosure is 1×10⁸ to 1×10¹⁵ vector genomes. In some cases, the unit dose of the pharmaceutical composition of the disclosure is at most about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ vector genomes. In some cases, the unit dose is between 10¹⁰ to 10¹¹, between 10¹¹ to 10¹², between 10¹⁰ to 10¹², between 10¹² to 10¹³, between 10¹¹ to 10¹³, between 10¹² to 10¹³, between 10¹² to 10¹⁴, between 10¹¹ to 10¹⁴, between 10¹¹ to 10¹⁵, between 10¹² to 10¹⁵, between 10¹³ to 10¹⁴, between 10¹⁴ to 10¹⁵, between 10¹⁵ to 10¹⁶, between 10¹⁶ to 10¹⁷, between 10¹⁷ to 10¹⁸, between 10¹⁸ to 10¹⁹, or between 10¹⁹ to 10²⁰ vector genomes.

In some cases, the unit dose of the pharmaceutical composition of the disclosure is between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 2×10¹¹ to 2.5×10¹¹, between 2.5×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹⁰, to 2×10¹², between 2×10¹² to 3×10¹², between 2.5×10¹² to 3×10¹², between 3×10¹² to 4×10¹², between 4×10¹² to 5×10¹², between 5×10¹² to 6×10¹², between 6× 10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes.

In some cases, the unit dose of rAAV of this disclosure is between 2×10¹¹ to 8×10¹¹ or between 2×10¹² to 8×10¹² vector genomes. In some cases, the unit dose of rAAV of this disclosure is between 10¹⁰ to 10¹³, between 10¹⁰ to 10¹¹, between 10¹¹ to 10¹², between 10¹² to 10¹³, or between 10¹³ to 10¹⁴ vector genomes.

In some cases, the unit dose of rAAV of this disclosure is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰ to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹³, between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 8×10¹³ to 1×10¹⁴ vector genomes.

In some cases, a lower amount or range of vector genomes is selected for a unit dose to avoid aggregation. In some cases, a higher amount or range of vector genomes is selected for a unit dose so that a smaller volume can be used for injection. Smaller volume (e.g., less than 50, 40, 30, 20, 10, or 5 µL) of injection can help to reduce changes in ocular pressure and other adverse effects associated with intravitreal injection. In some cases, a higher concentration of rAAV also helps to ensure efficient delivery of the therapeutic transgene into target cells.

In some cases, a unit dose comprise between 2E12 to 6E12 vector genomes. In some cases, a unit dose comprises about 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some cases, a unit dose comprises between 1E12 to 1.5E12, between 1.5E12 to 2E12, between 2E12 to 2.5E12, between 2.5E12 to 3.0E12, between 3.0E12 to 3.5E12, between 3.5E12 to 4.0E12, between 4.0E12 to 4.5E12, between 4.5E12 to 5.0E12, between 5.0E12 to 5.5E12, between 5.5E12 to 6.0E12, between 6.0E12 to 6.5E12, between 6.5E12 to 7.0E12, between 7.0E12 to 7.5E12, between 7.5E12 to 8.0E12, between 8.0E12 to 8.5E12, between 8.5E12 to 9.0E12, between 9.0E12 to 9.5E12, or between 9.5E12 to 10E12 vector genomes. In some cases, a unit dose comprises at least 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some cases, a unit dose comprises no more than 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes.

In some cases, the unit dose of the pharmaceutical composition of the disclosure can be measured using multiplicity of infection (MOI). In some cases, MOI can refer to the ratio, or multiple of vector or viral genomes to the cells to which the nucleic may be delivered. In some cases, the MOI can be 1×10⁶. In some cases, the MOI can be between about 1×10⁵ to about 1×10⁷. In some cases, the MOI may be 1×10⁴-1×10⁸. In some cases, recombinant viruses of the disclosure can be at least about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some cases, recombinant viruses of this disclosure can be from about 1×10⁸ to about 1×10¹⁵ MOI. In some cases, recombinant viruses of the disclosure can be at most about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some cases, the MOI is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰, to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹³, between 1×10¹³, to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 8×10¹³ to 1×10¹⁴.

Pharmaceutical compositions suitable for ocular use include sterile aqueous solutions or dispersions and sterile powders for the extemporaneous preparation of sterile injectable solutions, suspension, or dispersion. For intravitreal administration, suitable carriers include physiological saline, bacteriostatic water, phosphate buffered saline (PBS), and/or an isotonic agent, e.g., glycerol. In all cases, the pharmaceutical composition must be sterile and should be fluid to the extent that easy syringability or injectability exists. It must be stable under the conditions of manufacture and storage and must be preserved against the contaminating action of microorganisms such as bacteria and fungi. In some cases, the pharmaceutical composition can include an isotonic agent, such as a salt or glycerol. In some cases, a surfactant or a stabilizer is added to the pharmaceutical composition to prevent aggregation.

In some instances, the excipient can be a carrier. A carrier can be a solvent or dispersion medium containing, for example, water, saline, ethanol, a polyol (for example, glycerol, propylene glycol, and liquid polyethylene glycol, and the like), and any combination thereof. The proper fluidity can be maintained, for example, by the use of a coating such as lecithin, by the maintenance of the required particle size in the case of dispersion and by the use of surfactants such as polysorbates (e.g., Tween^{™}, polysorbate 20, polysorbate 80), sodium dodecyl sulfate (sodium lauryl sulfate), lauryl dimethyl amine oxide, cetyltrimethylammonium bromide (CTAB), polyethoxylated alcohols, polyoxyethylene sorbitan, octoxynol (Triton X100^{™}), N,N-dimethyldodecylamine-N-oxide, hexadecyltrimethylammonium bromide (HTAB), polyoxyl 10 lauryl ether, Brij 721^{™}, bile salts (sodium deoxycholate, sodium cholate), pluronic acids (F-68, F-127), polyoxyl castor oil (Cremophor^{™}) nonylphenol ethoxylate (Tergitol^{™}), cyclodextrins and, ethylbenzethonium chloride (Hyamine^{™}) Prevention of the action of microorganisms can be achieved by various antibacterial and antifungal agents, for example, parabens, chlorobutanol, phenol, cresol, ascorbic acid, thimerosal, and the like. In many cases, it will be preferable to include isotonic agents, for example, sugars, polyalcohols such as mannitol, sorbitol, sodium chloride in the composition. Prolonged absorption of the internal compositions can be brought about by including in the composition an agent which delays absorption, for example, aluminum monostearate and gelatin. In some cases, the pharmaceutical carrier includes sodium phosphate, sodium chloride, polysorbate, and sucrose. In some cases, a pharmaceutical composition comprises a surfactant, e.g., non-ionic surfactant such as polysorbate, poloxamer, or pluronic. In some cases, the addition of a non-ionic surfactant reduces aggregation in a suspension or solution.

In some cases, pharmaceutical compositions useful for the present disclosure can be packaged in a kit to facilitate application of the present disclosure. In some aspects, the present method provides for a kit comprising a recombinant nucleic (e.g., rAAV comprising the nucleic acid sequence of an anti-VEGF agent) of the disclosure. In some aspects, the present method provides for a kit comprising a lyophilized form of a recombinant virus of the disclosure and a solution for reconstituting the virus before administration to a patient. In some cases, the kit comprises a suspension form of the recombinant virus of the disclosure and a solution for diluting the suspension. In some cases, the suspension is supplied in as a pre-filled syringe. In some cases, the suspension or a kit thereof is refrigerated. In some cases, the suspension is warmed to room temperature before administration. In some cases, the suspension is agitated to ensure even distribution before administration.

In some cases, a kit comprises: a recombinant virus provided herein, and instructions to administer to an eye or retinal cells of a subject in a therapeutically effective amount of the recombinant virus. In some aspects, the kit comprises pharmaceutically acceptable salts or solutions for administering the recombinant virus. Optionally, the kit can further comprise instructions for suitable operational parameters in the form of a label or a separate insert. For example, the kit may have standard instructions informing a physician or laboratory technician to prepare a unit dose of recombinant virus from a solution or suspension and/or to reconstitute the lyophilized compositions. In some cases, optionally, the kit further comprises a device for administration, such as a syringe, filter needle, extension tubing, cannula, or subretinal injector.

In some cases, the pharmaceutical composition is provided as a refrigerated suspension. In some cases, the refrigerated suspension is provided in a kit, which can include a syringe and/or buffer for dilution. In some cases, the refrigerated suspension is provided as a pre-filled syringe.

In some cases, any suitable method can be used in the biochemical purification of recombinant viruses (e.g., rAAV) for use in a pharmaceutical composition as described herein. Recombinant AAV viruses can be harvested directly from cells, or from the culture media comprising cells. Virus can be purified using various biochemical means, such as gel filtration, filtration, chromatography, affinity purification, gradient ultracentrifugation, or size exclusion methods before lyophilizing or making a suspension of the rAAV viruses.

### Indications

In some cases, rAAV virion of any serotype comprising a variant capsid protein and a therapeutic transgene, or a pharmaceutical composition thereof as described herein, can at least partially ameliorate an eye condition or disease associated with neovascularization of the eye, or associated with CNV. In some cases, a rAAV virion comprising a capsid variant protein is used to deliver an anti-VEGF transgene into an eye of a human subject.

Indications gene therapy or pharmaceutical compositions described herein include neovascular (wet) age-related macular degeneration (AMD), macular edema following retinal vein occlusion (RVO), diabetic macular edema (DME), retinal vein occlusion, and diabetic retinopathy (DR) in patients with DME. In some cases, methods and pharmaceutical compositions disclosed herein can be used to prevent or treat an eye condition or disease for which an anti-VEGF transgene is approved or indicated for. In some cases, a gene therapy (e.g., AAV2.7m8 based gene therapy) is used to treat or prevent an eye condition or disease that is responsive to at least one current standard of care for the eye condition/disease, including, but not limited to, CNV, wet AMD, dry AMD, macular edema following RVO, DME, and diabetic retinopathy in patients with DME. In some cases, a rAAV gene therapy is used to treat or prevent any eye condition or disorder characterized by neovascularization or CNV. In another aspect, the present disclosure provides pharmaceutical compositions provided herein for the treatment of diseases such as, for example: AMD, DME, RVO, angiogenesis related diseases, cancer, autoimmune diseases, infectious disease organisms, and the like.

In some cases, the eye condition can be diabetic macular edema. Diabetic macular edema (DME) is a swelling of the retina in diabetes mellitus due to leaking of fluid from blood vessels within the macula. The macula is the central portion of the retina, a small area rich in cones, the specialized nerve endings that detect color and upon which daytime vision depends. As macular edema develops, blurring occurs in the middle or just to the side of the central visual field. Visual loss from diabetic macular edema can progress over a period of months and make it impossible to focus clearly. Common symptoms of DME are blurry vision, floaters, double vision, and eventually blindness if it goes untreated. In some cases, methods and pharmaceutical compositions as disclosed herein are used to treat DME

In some cases, the eye condition can be a retinal vein occlusion. Retinal vein occlusion is a blockage of the small veins that carry blood away from the retina. The retina is the layer of tissue at the back of the inner eye that converts light images to nerve signals and sends them to the brain. Retinal vein occlusion is most often caused by hardening of the arteries (atherosclerosis) and the formation of a blood clot. Blockage of smaller veins (branch veins or BRVO) in the retina often occurs in places where retinal arteries that have been thickened or hardened by atherosclerosis cross over and place pressure on a retinal vein. Symptoms of retinal vein occlusion can include a sudden blurring or vision loss in all or part of one eye. In some cases, methods and pharmaceutical compositions as disclosed herein are used to treat retinal vein occlusion.

In some cases, the eye condition can be choroidal neovascularization (CNV), also known as wet AMD. Choroidal neovascularization can involve the growth of new blood vessels that originate from the choroid through a break in the Bruch membrane into the sub-retinal pigment epithelium (sub-RPE) or subretinal space, which can be a major cause of visual loss. CNV can create a sudden deterioration of central vision, noticeable within a few weeks. Other symptoms which can occur include color disturbances, and metamorphopsia (distortions in which straight lines appears wavy). Hemorrhaging of the new blood vessels can accelerate the onset of symptoms of CNV. CNV may also include the feeling of pressure behind the eye. In some cases, methods and pharmaceutical compositions as disclosed herein are used to treat CNV or an eye condition associated with neovascularization.

The advanced "wet" form (neovascular or exudative) of AMD is less common, but may frequently cause a rapid and often substantial loss of central vision in patients. In the wet form of AMD, choroidal neovascularization forms and develops into a network of vessels that may grow under and through the retinal pigment epithelium. As this is accompanied by leakage of plasma and/or hemorrhage into the subretinal space, there could be severe sudden loss of central vision if this occurs in the macula. The term "AMD", if not otherwise specified, can be either dry AMD or wet AMD. The present disclosure contemplates treatment or prevention of AMD, wet AMD and/or dry AMD. In some cases, methods and pharmaceutical compositions as disclosed herein are used to treat AMD

In some cases, methods and pharmaceutical compositions as disclosed herein are used to prevent or treat an eye disease or condition that is responsive to at least one of the current standard of care or approved therapies, such as ranibizumab or bevacizumab. In some cases, a patient has been pre-treated with any one of ranibizumab, bevacizumab, and any other approved therapeutics for the eye disease or condition, or any combination thereof, before receiving or qualifying for an administration of an anti-VEGF gene therapy.

In some cases, methods and pharmaceutical compositions disclosed herein, i.e., AAV gene therapy comprising an anti-VEGF agent, results in a reduction in neovascularization or CNV, as measured by percentage of grade IV lesions following CNV formation according to color fundus photography, by at least 5%, at least 6%, at least7%, at least 8%, at least 9%, at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% as compared to a vehicle or buffer control.

In some cases, methods and pharmaceutical compositions disclosed herein, i.e., AAV gene therapy comprising an anti-VEGF agent, results in a reduction in neovascularization or CNV, as measured by percentage of grade IV lesions following CNV formation according to color fundus photography, that is comparable to an approved therapy. In some cases, the reduction in CNV, or the therapeutic effect, lasts longer with the administration of a gene therapy comprising an anti-VEGF agent as compared to a non-gene therapy-based injection or a protein injection.

In some cases, a rAAV virion or pharmaceutical composition thereof can at least partially ameliorate an eye condition, disease, or combinations thereof. In some instances, the eye condition or disease can be associated with neovascularization of the eye. In some cases, the eye condition or disease is any condition or disease responsive to or treatable with an anti-VEGF agent of the present disclosure.

In some cases, a gene therapy as described herein is used to treat any eye disease or condition involving abnormal neovascularization, e.g., as a result of abnormal VEGF and/or VEGFR activity or expression, AMD, diabetic retinopathy, and preeclampsia. In some cases, an anti-VEGF agent in a gene therapy is an agent that inhibits or interferes with a member of the VEGF family in mammals, which includes VEGF-A, B, C, D, and placenta growth factor (PIGF), or any combination or variant thereof. In some cases, an anti-VEGF agent in a gene therapy is an agent that inhibits or interferes with any one of the VEGF-related proteins, e.g.,VEGF-E expressed by some viruses and VEGF-F found in venom of some snakes, which may also have therapeutic properties for additional indications related to angiogenesis in vivo. In some cases, the anti-VEGF agent can also interfere with, bind, or inhibit placental growth factor (PIGF) in vivo.

### Methods of use

In some cases, present disclosure provides a method for treating a pathological angiogenesis related eye disease, comprising administering a pharmaceutically effective amount of the pharmaceutical compositions provided herein to a human subject in need of such treatment. In some cases, the disease is selected from the group of ocular neovascular diseases including age-related macular degeneration (AMD), wet-AMD, dry-AMD, retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy and diabetic retinal edema, and any combination thereof.

In some cases, pharmaceutical compositions comprising a rAAV comprising a variant capsid protein (e.g., rAAV.7m8) and a nucleic acid sequence that encodes an anti-VEGF agent is used to treat or prevent AMD, including dry AMD and wet AMD. In some cases, pharmaceutical compositions comprising a rAAV comprising a variant capsid protein (e.g., rAAV.7m8) and a nucleic acid sequence that encodes an anti-VEGF agent is used to treat or prevent CNV, or reduce grade IV CNV lesions. In some cases, pharmaceutical compositions comprising a rAAV comprising a variant capsid protein (e.g., rAAV.7m8) and a nucleic acid sequence that encodes an anti-VEGF agent is used to treat or prevent any one of AMD, wet-AMD, dry-AMD, retinal neovascularization, choroidal neovascularization diabetic retinopathy, proliferative diabetic retinopathy, retinal vein occlusion, central retinal vein occlusion, branched retinal vein occlusion, RVO, diabetic macular edema, diabetic retinal ischemia, ischemic retinopathy and diabetic retinal edema, DR in patients with DME, and any combination thereof.

In some cases, the method of treating AMD, DME, RVO, or DR comprises pre-treating a patient with an approved therapy, e.g., ranibizumab or bevacizumab injection, before administering a gene therapy comprising a nucleic acid sequence of the anti-VEGF agent, e.g., ranibizumab or bevacizumab, to the same patient. In some cases, a patient is pre-treated with an approved therapy before receiving a one-time dose of the anti-VEGF gene therapy, as disclosed herein. In some cases, a patient is responsive to any one of ranibizumab or bevacizumab injection before receiving a one-time dose of the anti-VEGF gene therapy, as disclosed herein. In some cases, a patient who is responsive to any one of ranibizumab or bevacizumab, or who was pre-treated with one of ranibizumab or bevacizumab, is treated with ranibizumab or bevacizumab gene therapy, as disclosed herein, followed by a period of at least 1.5, 2, 3, 4, 5, 6, 7, 8, 9, 10 or more years, or more than 1.5, 2, 3, 4, 5, 6, 7, 8, 9, 10 or more years during which the patient does not receive any of these treatment for AMD. In some cases, after a patient receives an intravitreal injection of ranibizumab or bevacizumab gene therapy, the patient does not begin receiving ranibizumab or bevacizumab protein injection or another approved therapy until at least 1.5, 2, 3, 4, 5, 6, 7, 8, 9, 10 or more years have lapsed.

In some cases, ranibizumab or bevacizumab gene therapy, or any other anti-VEGF gene therapy, as disclosed herein is a one-time administration. In some cases, after a patient receives a unit dose of ranibizumab or bevacizumab gene therapy disclosed herein, the patient does not need to use any other approved protein-based therapeutics.

In some cases, patients who experience adverse effects associated with repeated injections of approved therapies for CNV or AMD, e.g., inflammation or bacterial infection, can be candidates for treatment with the anti-VEGF gene therapy, or ranibizumab or bevacizumab gene therapy, as disclosed herein. In some cases, such risks are lower in gene therapy because it requires only one injection in a patient's lifetime, or is given not more than once in at least 2, 5, 10, 20, 30, 40, or 50 years. In some cases, treatment with the anti-VEGF gene therapy, or ranibizumab or bevacizumab gene therapy, as disclosed herein can be more cost-effective than protein-based injections because a gene therapy's therapeutic effects can last longer and the cost of a one-time gene therapy injection may be lower than the combined cost of multiple, repeated injections of a protein.

Also, by not requiring repeated injections, gene therapy addresses the patient compliance and adherence challenge associated with therapies that require repeated injections, as non-compliance (e.g., when a patient forgets or misses one or more scheduled injection) can result in vision loss and deterioration of the eye disease or condition. The rate of non-compliance and non-adherence to treatment regimens that require repeated or frequent trips to medical offices for administration is higher among elderly patients, who are most impacted by AMD. Therefore, delivering an anti-VEGF agent into an eye of a patient via gene therapy, e.g., as a one-time intravitreal injection, can provide a more convenient treatment option for patients and improve patient outcomes by addressing the non-compliance and non-adherence problem.

In some cases, a method of use comprises pre-treating a human patient or subject with an approved drug that is considered the current standard of care, e.g., ranibizumab injection, or bevacizumab injection, determining the patient's responsiveness to ranibizumab or bevacizumab, and administering the anti-VEGF gene therapy described herein to the patient who is responsive to an approved therapy. Determining a patient's responsiveness to an approved therapy or a current standard of care can include, but not limited to, blood tests, immunoassay, ex vivo experiments, or administration of the ranibizumab or bevacizumab protein injection to the patient and assaying the patient's responsiveness to ranibizumab or bevacizumab.

In some cases, method of use of the anti-VEGF gene therapy described herein includes reconstituting a lyophilized form of the pharmaceutical composition described herein (i.e., rAAV2.7m8 comprising an anti-VEGF nucleic acid sequence) according to the drug label and administering said reconstituted anti-VEGF gene therapy to a subject or human patient. In some cases, method of use of the anti-VEGF gene therapy described herein includes administering a suspension of the pharmaceutical composition described herein according to the drug label and administering said suspension of anti-VEGF gene therapy to a subject or human patient. In some cases, additional steps for administering a suspension include agitating the suspension before use and/or warming the suspension to room temperature.

In some cases, such human patient was pre-treated with an approved protein injection or current standard of care, e.g., ranibizumab injection or bevacizumab injection. In some cases, such patient receives no more than one injection or administration of the rAAV2.7m8-ranibizumab gene therapy for at least 2, 3, 4, 5, 6, 7, 8, 9, 10 or more years; or receives no more than one injection or administration of the rAAV2.7m8-ranibizumab gene therapy in more than 2, 3, 4, 5, 6, 7, 8, 9, 10 or more years.

In some cases, also disclosed herein are methods of preventing or treating an eye condition or disease, the method comprising administering to an individual in need thereof, e.g., an individual with an eye condition or disease responsive to an approved drug, an effective amount of a rAAV virion comprising a nucleic acid sequence that encodes an anti-VEGF agent, e.g., ranibizumab or bevacizumab, as described herein or a pharmaceutical composition thereof. In some cases, rAAV2.7m8- ranibizumab virion can be administered via intraocular injection, by intravitreal injection, by subretinal injection, or by any other convenient mode or route of administration into an eye of an individual. Other convenient modes or routes of administration can include, e.g., intravenous, topical, eye drops, etc. In some cases, methods and pharmaceutical compositions disclosed herein involve administration by intravitreal injection.

A "therapeutically effective amount" as described herein can be a relatively broad range that can be determined through clinical trials. For injection directly into the eye or intravitreal injection, a therapeutically effective dose can be on the order of from 10¹¹ to 10¹² or from 10¹² to 10¹³ vector genomes of 7m8-ranibizumab or any other anti-VEGF gene therapy. In some cases, the unit dose or a therapeutically effective amount of 7m8-ranibizumab or any other anti-VEGF gene therapy is between 10¹⁰ to 10¹¹, between 10¹¹ to 10¹², between 10¹⁰ to 10¹², between 10¹² to 10¹³, between 10¹¹ to 10¹³, between 10¹² to 10¹³, between 10¹² to 10¹⁴, between 10¹¹ to 10¹⁴, between 10¹¹ to 10¹⁵, between 10¹² to 10¹⁵, between 10¹³ to 10¹⁴, between 10¹⁴ to 10¹⁵, between 10¹⁵ to 10¹⁶, between 10¹⁶ to 10¹⁷, between 10¹⁷ to 10¹⁸, between 10¹⁸ to 10¹⁹, or between 10¹⁹ to 10²⁰ vector genomes. In some cases, the unit dose of the pharmaceutical composition comprising 7m8-ranibizumab or any other anti-VEGF gene therapy of the disclosure is between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 2×10¹¹ to 2.5×10¹¹, between 2.5×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹² to 2×10¹², between 2×10¹² to 3×10¹⁰, between 2.5×10¹² to 3×10¹², between 3×10¹² to 4×10¹⁰, between 4×10¹² to 5×10¹², between 5×10¹² to 6×10¹², between 6×10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹⁰, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes. In some cases, the unit dose of 7m8- ranibizumab or any other anti-VEGF gene therapy of this disclosure is between 2.1×10¹¹ or between 2.1×10¹² vector genomes. In some cases, the unit dose of rAAV of this disclosure is between 10¹⁰ to 10¹³, between 10¹⁰ to 10¹¹, between 10¹¹ to 10¹², between 10¹² to 10¹³, or between 10¹³ to 10¹⁴ vector genomes.

In some cases, the unit dose of 7m8- ranibizumab or any other anti-VEGF gene therapy of this disclosure is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰ to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹⁰, between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹⁰, or between 8×10¹³ to 1×10¹⁴ vector genomes.

In some cases, the total amount of 7m8-ranibizumab or any other anti-VEGF gene therapy injected into a human patient or subject within a period of 5 to 10 years is no more than 10¹⁰ to 10¹³, 10¹⁰ to 10¹¹, 10¹¹ to 10¹², 10¹² to 10¹³, or 10¹³ to 10¹⁴ vector genomes, or no more than 1×10¹⁰ to 2×10¹⁰, 2×10¹⁰ to 4×10¹⁰, 3×10¹⁰ to 5×10¹⁰, 4×10¹⁰ to 6×10¹⁰, 5×10¹⁰ to 7× 10¹⁰, 6×10¹⁰ to 8×10¹⁰, 7×10¹⁰ to 9×10¹⁰, 8×10¹⁰ to 10¹¹, 1×10¹¹ to 2×10¹¹, 2×10¹¹ to 4×10¹¹, 3×10¹¹ to 5×10¹¹, 4×10¹¹ to 6×10¹¹, 5×10¹¹ to 7×10¹¹, 6×10¹¹ to 8×10¹¹, 7×10¹¹ to 9×10¹¹, 8×10¹¹ to 10×10¹¹, 1×10¹² to 3×10¹², 2×10¹² to 4×10¹², 3×10¹² to 5×10¹², 4×10¹² to 6×10¹², 5×10¹² to 7×10¹², 6×10¹² to 8×10¹², 7×10¹² to 9×10¹², 8×10¹² to 10×10¹², 1×10¹³ to 5×10¹³, 5×10¹³ to 10×10¹³, 10¹² to 5×10¹², 5×10¹² to 1×10¹³, 7×10¹² to 1×10¹³, 8×10¹² to 2×10¹³, 9×10¹² to 2×10¹³, 9×10¹² to 2×10¹³, 9×10¹² to 4×10¹³, 1×10¹³ to 3×10¹³, 1×10¹³ to 2×10¹³, 2×10¹³ to 3×10¹³, 3×10¹³ to 4×10¹³, 4×10¹³ to 5×10¹³, 5×10¹³ to 6×10¹³, 6×10¹³ to 7×10¹³, 7×10¹³ to 8×10¹³, 8×10¹³ to 9×10¹⁰, or 8×10¹³ to 1×10¹⁴ vector genomes.

In some cases, the therapeutically effective amount of pharmaceutical compositions disclosed herein comprises between 2E12 to 6E12 vector genomes. In some cases, a unit dose comprises about 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some cases, a unit dose comprises between 1E12 to 1.5E12, between 1.5E12 to 2E12, between 2E12 to 2.5E12, between 2.5E12 to 3.0E12, between 3.0E12 to 3.5E12, between 3.5E12 to 4.0E12, between 4.0E12 to 4.5E12, between 4.5E12 to 5.0E12, between 5.0E12 to 5.5E12, between 5.5E12 to 6.0E12, between 6.0E12 to 6.5E12, between 6.5E12 to 7.0E12, between 7.0E12 to 7.5E12, between 7.5E12 to 8.0E12, between 8.0E12 to 8.5E12, between 8.5E12 to 9.0E12, between 9.0E12 to 9.5E12, or between 9.5E12 to 10E12 vector genomes. In some cases, a unit dose comprises at least 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some cases, a unit dose comprise no more than 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, 9.5E12, or 10E12 vector genomes.

In some cases, a lower concentration (e.g., vector genomes) is used for a unit dose to prevent aggregation, which can occur at higher concentrations. In some cases, a higher concentration, e.g., higher vector genomes, is selected for a unit dose to increase efficacy of the gene therapy, or to maximize the delivery of the anti-VEGF transgene in one injection or in a one-time administration of the gene therapy. In some cases, higher concentrations of the pharmaceutical compositions disclosed herein allow smaller volumes of injection, which can reduce adverse effects associated with intravitreal injection, e.g., elevated intraocular pressure, inflammation, irritation, or pain.

In some cases, 7m8-ranibizumab or any other anti-VEGF gene therapy or a pharmaceutical composition thereof can be administered as a single dose or a one-time dose. In some cases, more than one administration may be employed to achieve the desired level of gene expression over a sustained period of various intervals, e.g., not more than once in at least 2 years, or at least 3, 4, 5, 6, 7, 8, 9, 10, or more years. In some cases, intravitreal injection of 7m8-ranibizumab or any other anti-VEGF gene therapy obviates a patient's need to receive an approved protein injection for at least 1 year or 2, 3, 4, 5, 6, 7, 8, 9, 10, 20, 30 or more years.

### EXAMPLES

### Example 1: Efficacy evaluation of 7m8-sVEGFR-1 in monkeys

Objective: To assess the efficacy of 7m8-sFLT-1 following intravitreal (IVT) administration at 2 × 10¹² vg to inhibit the development of choroidal neovascularization (CNV) induced by laser photocoagulation in African green monkeys. An additional objective can be to evaluate regional sFLT-1 expression in ocular tissues.

CNV lesion model in monkeys is a generally accepted as and a widely used standard primate model for assessing potential efficacy of therapies for treating eye diseases associated with neovascularization, such as wet AMD.

Subject Recruitment: Monkeys underwent baseline screening to assess ocular and general health by tonometry, slit lamp biomicroscopy, fundoscopy, color fundus photography (CFP), fluorescence angiography (FA) and optical coherence tomography (OCT). Thirty-nine animals with normal findings were enrolled in the study and randomized into four treatment groups by baseline body weight and gender **(Table 1).** Atropine 1% ophthalmic ointment was applied following baseline exam.

**Table 1: Treatment Assignment**

| **Group** | **N** | **Treatment OU** | **Route** | **Dose (µL)** | **Laser OU** | **Slit lamp & CFP** | **FA & OCT** | **Terminus & tissue collection** |
|---|---|---|---|---|---|---|---|---|
| 1 | 6 | AAV2.7m8-sVEGFR-1 | IVT; Day 0 | 1×100µ L | Day 56 | Baseline, days 0 (post-injection), 7, 14, 56 and 84 | Baseline, post-bleb, day 70 & 84 | Day 85 |
| 2 | 6 | Vehicle | IVT; Day 0 | 1×100µ L | Day 56 | | | |

| | | | | | | | | |
|---|---|---|---|---|---|---|---|---|
| ^{∗} CFP will be additionally performed on day 21 if day 14 images do not reveal clear images of stabilized blebs. Slit lamp was performed prior to laser on day 56 but not immediately post-injection on day 0. | | | | | | | | |

On study day 0 groups 1-2 monkeys received IVT AAV2.7m8-sFLT-1 or vehicle OU in accordance with the treatment schedule (**Table 1**). Prior to IVT dosing, topical local anesthesia was administered (0.5% proparacaine) and eyes were disinfected with 5% Betadine and rinsed with sterile normal saline. IVT injections can be administered using a 31-gauge 0.5-inch needle placed 2 mm posterior to the limbus in the inferior temporal quadrant, targeting the central vitreous.

All IVT injections can be followed by topical administration of 0.3% ciprofloxacin, or equivalent antibiotic ophthalmic solution, and 1% atropine sulfate ointment.

On Day 56, CNV was induced between temporal vascular arcades with laser bums. Nine laser spots were symmetrically placed in each eye by an ophthalmologist employing an Iridex Oculight TX 532 nm laser with a laser duration of 100 ms, spot size 50 µm, power 750 mW. Laser spots were applied using a 0.9× contact laser lens. The target location of laser spots were mapped by a trained ophthalmologist on color fundus images obtained prior to laser treatment (and subsequent to bleb placement) for reference during laser spot placement. Color fundus photography was performed immediately after the laser treatment to document the laser lesions. Any spots demonstrating severe retinal/subretinal hemorrhage immediately post-laser was excluded from analyses. **FIG. 1** illustrates an exemplary fundus photograph of an eye of a non-human primate after induction of CNV lesions by laser irradiation.

Bilateral color fundus images of the retina were captured with 50 degree of view centered on the fovea using a Topcon TRC-50EX retinal camera with Canon 6D digital imaging hardware and New Vision Fundus Image Analysis System software. FA was performed with intravenous administration of 0.1 mL/kg of 10% sodium fluorescein. Fluorescein leakage in angiograms of CNV lesions was graded (I-IV; **Table 2** by a masked ophthalmologist assessing composites generated after uniform adjustment of image intensity. Lesion grading assessment was confirmed on images of fundus by two other trained ophthalmologists. Image fluorescence densitometry analysis of late-stage raw angiograms can also be performed using ImageJ software.

**Table 2: Laser lesion grading scales**

| Lesion Grade | Definition |
|---|---|
| I | No hyperfluorescence - Compare pre-FA with 30 sec post-FA. Look for absence of hyperfluorescence in lesion |
| II | Hyperfluorescence without leakage - Compare 30 sec FA with 3 and 6 min FA. Look for hyperfluoresence without significant residual staining in 6 min FA. |
| III | Hyperfluorescence early or mid-transit and late leakage - Compare 30 sec FA with 3 and 6 min FA. Look for significant residual staining in lesion at 6 min FA. |
| IV | Hyperfluorescence early or mid-transit and late leakage extending beyond the borders of the treated area - Compare 30 sec FA with 3 and 6 min FA. Look for consistent staining beyond the border of the lesion as seen in 30 sec FA. |

Subjects were assessed twice daily for general wellbeing. Detailed observations were performed once weekly. Body weights were obtained at the time of baseline screening and every two weeks during the in-life study.

All animals were euthanized with pentobarbital after confirming the quality of fundus imaging on Day 85, or shortly thereafter, pending review of images. Animals were then be euthanized with pentobarbital and globes enucleated. Excess orbital tissue was trimmed and both OD and OS globes was flash frozen in liquid nitrogen then dissected along frozen tissue planes at room temperature to isolate vitreous and retinal with choroidal sub-tissues. After collection of vitreous, 5 mm punches of neural retina with RPE/choroid were taken from the macula and superior, inferior, temporal and nasal regions. As space permits, additional peripheral punches were made. The retina with underlying RPE/choroidal tissues from each punch was transferred to pre-tared labeled cryotubes, and weighed and flash frozen in liquid nitrogen. Before and after collection of the punch biopsies, a photograph of the flat mounted retina was taken with indication of orientation to document the regions from which the punches were collected.

Statistical methods: A Fisher's exact test was used to evaluate incidence of different lesion grades. A two way ANOVA with repeated measures followed by Tukey-Kramer test or a contrast procedure was used to analyze the OCT CNV complex area and angiogram image densitometry data. Non-parametric tests were applied if the data is not normally distributed and has an unequal variance. P value of 0.05 or less was considered statistically significant.

**FIG.** 3 illustrates a plot of the percentage of grade IV lesions on days 14 and 28 of animals of groups 3 and 4, injected intravitreally with either AAV2.7m8- sVEGFR-1 or a vehicle control comprising formulation buffer only. CNV lesions were induced by laser irradiation immediately after injection in each group of test subjects, and color fundus photography was used to grade each lesion on a scale of I-IV. Monkeys treated with AAV2.7m8- sVEGFR-1 showed a slight decrease in the amount of grade IV lesions compared to administration of vehicle alone for the fundus images collected on day 14 when administered intravitreally. Monkeys treated with intravitreal AAV2.7m8-sFLT-1 showed no significant decrease in in the amount of grade IV lesions compared to administration of vehicle alone at day 28.

### Example 2: Efficacy evaluation of 7m8-ranibizumab in monkeys

Similar in vivo studies as described in Example 1 were performed in monkeys using the same protocol and AAV2.7m8- ranibizumab, which is rAAV2 comprising the 7m8 sequence inserted between positions 587 and 588 of capsid protein VP1 of AAV2 and a nucleic acid sequence that encodes ranibizumab.

As illustrated in **FIG. 4**, AAV2.7m8-ranibizumab administered intravitreally prevented the occurrence of laser-induced grade IV CNV lesions. AAV2.7m8-ranibizumab, ranibizumab alone (positive control), or vehicle control comprising formulation buffer were administered to eyes of non-human primates via intravitreal injection at a dose of 2 × 10¹² vg. CNV lesions were then induced by laser irradiation in all groups, and color fundus photography was used to grade each lesion on a scale of I-IV. Measurements of percentage of grade IV lesions were then averaged and plotted. AAV2.7m8-ranibizumab significantly reduced CNV lesions in vivo to levels comparable to ranibizumab alone at day 14 (light gray bar) and at day 28 (dark gray bar).

These in vivo studies in monkeys suggested AAV2.7m8-ranibizumab can be a viable gene therapy option for humans.

## Claims

1. A pharmaceutical composition comprising:
(a) a rAAV2 variant comprising an amino acid sequence LGETTRP inserted between positions 587 and 588 of capsid protein VP1, a nucleic acid encoding: (i) a sequence having at least 95% identity to SEQ ID NO: 9; and (ii) a sequence having at least 95% identity to SEQ ID NO: 10, and
(b) a pharmaceutically acceptable excipient;
for use in method of treating an eye disease or condition selected from: neovascular (wet) age-related macular degeneration (AMD), choroidal neovascularization, macular edema following retinal vein occlusion, diabetic macular edema (DME), retinal vein occlusion, and diabetic retinopathy associated with DME, the method comprising administering a unit dose of the pharmaceutical composition by intravitreal injection to an eye of a primate subject in need thereof.

2. The composition for use according to claim 1, wherein the unit dose is between 1E8 to 3E14 vector genomes.

3. The composition for use according to claim 1, wherein the unit dose is between 1E9 to 3E13 vector genomes.

4. The composition for use according to claim 1, wherein the unit dose is between 1E10 to 1E13 vector genomes.

5. The composition for use according to claim 1, wherein the unit dose is between 2E12 to 6E12 vector genomes.

6. The composition for use according to any one of claims 1-5, wherein the subject is a human.

7. The composition for use according to any one of claims 1-6, wherein the eye condition or disease is choroidal neovascularization or wet AMD.

8. The composition for use according to any one of claims 1-7, wherein administering the composition results in a reduction in percentage of grade IV lesions by at least 5%, or by at least 10%, as compared to a vehicle control, as measured by color fundus photography.

9. The composition for use according to any one of the preceding claims, wherein the unit dose: (a) has a volume that is not more than 100 µL; or (b) has a volume that is not more than 50 µL.

10. The composition for use according to any one of the preceding claims, wherein the subject is responsive to at least one of ranibizumab, bevacizumab, and sVEGFR-1.

11. The composition for use according to any one of the preceding claims, wherein the subject has been pre-treated with ranibizumab or bevacizumab.

12. The composition for use according to any one of the preceding claims, wherein: (a) the administering by injection occurs not more than once in at least 2 years or not more than once in at least 5 years; or (b) the administering is a one-time administration.

13. The composition for use according to any one of the preceding claims, wherein the pharmaceutical composition is a suspension.

14. The composition for use according to claim 13, further comprising: (a) agitating the suspension to ensure even distribution prior to the administering step; and/or (b) warming the suspension to room temperature prior to the administering step.

15. The composition for use according to claim 13 or claim 14, wherein the suspension further comprises: (a) a surfactant, optionally wherein the surfactant is selected from polysorbates, sodium dodecyl sulfate, sodium lauryl sulfate, lauryl dimethyl amine oxide, polyethoxylated alcohols, polyoxyethylene sorbitan, octoxynol, Brij, pluronic, and polyoxyl castor oil; and/or (b) phenol, mannitol, sorbitol, or sodium chloride.

16. The composition for use according to any one of the preceding claims, further comprising administering an antibiotic solution, for example an antibiotic solution comprising ciprofloxacin, or an atropine sulfate ointment after the injection.

17. The composition for use according to any one of the preceding claims, wherein the rAAV2 variant comprises a nucleic acid encoding the sequence of SEQ ID NO: 9 and the sequence of SEQ ID NO: 10.

## Patentansprüche

1. Pharmazeutische Zusammensetzung, die Folgendes umfasst:
(a) eine rAAV2-Variante, die eine Aminosäuresequenz LGETTRP umfasst, die zwischen den Positionen 587 und 588 des Kapsidproteins VP1 eingefügt ist, wobei eine Nukleinsäure für Folgendes kodiert: (i) eine Sequenz, die wenigstens 95 % Identität zu SEQ ID NO: 9 aufweist; und (ii) eine Sequenz, die wenigstens 95 % Identität zu SEQ ID NO: 10 aufweist, und
(b) einen pharmazeutisch unbedenklichen Arzneistoffträger;
zur Verwendung in einem Verfahren zur Behandlung einer Augenerkrankung oder eines -leidens, ausgewählt aus: neovaskulärer (feuchter) altersbedingter Makuladegeneration (AMD), choroidaler Neovaskularisation, Makulaödem nach Netzhautvenenverschluss, diabetischem Makulaödem (diabetic macular edema - DME), Netzhautvenenverschluss und diabetischer Retinopathie im Zusammenhang DME, wobei das Verfahren ein Verabreichen einer Einheitsdosis der pharmazeutischen Zusammensetzung durch intravitreale Injektion in ein Auge eines Primatensubjekts, das dies benötigt, umfasst.

2. Zusammensetzung zur Verwendung nach Anspruch 1, wobei die Einheitsdosis zwischen 1E8 bis 3E14 Vektorgenome beträgt.

3. Zusammensetzung zur Verwendung nach Anspruch 1, wobei die Einheitsdosis zwischen 1E9 bis 3E13 Vektorgenome beträgt.

4. Zusammensetzung zur Verwendung nach Anspruch 1, wobei die Einheitsdosis zwischen 1E10 bis 1E13 Vektorgenome beträgt.

5. Zusammensetzung zur Verwendung nach Anspruch 1, wobei die Einheitsdosis zwischen 2E12 bis 6E12 Vektorgenome beträgt.

6. Zusammensetzung zur Verwendung nach einem der Ansprüche 1-5, wobei das Subjekt ein Mensch ist.

7. Zusammensetzung zur Verwendung nach einem der Ansprüche 1-6, wobei das Augenleiden oder die -erkrankung eine choroidale Neovaskularisation oder feuchte AMD ist.

8. Zusammensetzung zur Verwendung nach einem der Ansprüche 1-7, wobei das Verabreichen der Zusammensetzung zu einer Verringerung eines Prozentsatzes von Läsionen des Grades IV um wenigstens 5 % oder um wenigstens 10 %, verglichen mit einer Trägerstoffkontrolle, wie durch Farbfundusfotografie gemessen, führt.

9. Zusammensetzung zur Verwendung nach einem der vorhergehenden Ansprüche, wobei die Einheitsdosis: (a) ein Volumen aufweist, das nicht mehr als 100 µl beträgt; oder (b) ein Volumen aufweist, das nicht mehr als 50 µl beträgt.

10. Zusammensetzung zur Verwendung nach einem der vorhergehenden Ansprüche, wobei das Subjekt auf Ranibizumab, Bevacizumab und/oder sVEGFR-1 anspricht.

11. Zusammensetzung zur Verwendung nach einem der vorhergehenden Ansprüche, wobei das Subjekt mit Ranibizumab oder Bevacizumab vorbehandelt wurde.

12. Zusammensetzung zur Verwendung nach einem der vorhergehenden Ansprüche, wobei:
(a) das Verabreichen durch Injektion nicht öfter als einmal in wenigstens 2 Jahren oder nicht öfter als einmal in wenigstens 5 Jahren erfolgt; oder (b) das Verabreichen eine einmalige Verabreichung ist.

13. Zusammensetzung zur Verwendung nach einem der vorhergehenden Ansprüche, wobei die pharmazeutische Zusammensetzung eine Suspension ist.

14. Zusammensetzung zur Verwendung nach Anspruch 13, die ferner Folgendes umfasst: (a) Hin- und herbewegen der Suspension, um eine gleichmäßige Verteilung vor dem Verabreichungsschritt sicherzustellen; und/oder (b) Erwärmen der Suspension auf Raumtemperatur vor dem Verabreichungsschritt.

15. Zusammensetzung zur Verwendung nach Anspruch 13 oder 14, wobei die Suspension ferner Folgendes umfasst: (a) ein Tensid, optional wobei das Tensid aus Polysorbaten, Natriumdodecylsulfat, Natriumlaurylsulfat, Lauryldimethylaminoxid, polyethoxylierten Alkoholen, Polyoxyethylensorbitan, Octoxynol, Brij, Pluronic und Polyoxylrizinusöl ausgewählt ist; und/oder (b) Phenol, Mannit, Sorbit oder Natriumchlorid.

16. Zusammensetzung zur Verwendung nach einem der vorhergehenden Ansprüche, die ferner das Verabreichen einer antibiotischen Lösung, beispielsweise einer antibiotischen Lösung, die Ciprofloxacin umfasst, oder einer Atropinsulfatsalbe nach der Injektion umfasst.

17. Zusammensetzung zur Verwendung nach einem der vorhergehenden Ansprüche, wobei die rAAV2-Variante eine Nukleinsäure umfasst, die für die Sequenz von SEQ ID NO: 9 und die Sequenz von SEQ ID NO: 10 kodiert.

## Revendications

1. Composition pharmaceutique comprenant :
(a) un variant rAAV2 comprenant une séquence d'acides aminés LGETTRP insérée entre les positions 587 et 588 de la protéine de capside VP1, un acide nucléique codant : (i) une séquence ayant au moins 95 % d'identité avec SEQ ID NO : 9 ; et (ii) une séquence ayant au moins 95 % d'identité avec SEQ ID NO : 10, et
(b) un excipient pharmaceutiquement acceptable ;
destinée à être utilisée dans un procédé de traitement d'une maladie ou affection oculaire choisie parmi : la dégénérescence maculaire liée à l'âge (DMLA) néovasculaire (humide), la néovascularisation choroïdienne, l'œdème maculaire après occlusion de la veine rétinienne, l'œdème maculaire diabétique (DME), l'occlusion veineuse de la rétine et la rétinopathie diabétique associée au DME, le procédé comprenant l'administration d'une dose unitaire de la composition pharmaceutique par injection intravitréenne à un œil d'un sujet primate en ayant besoin.

2. Composition destinée à être utilisée selon la revendication 1, dans laquelle la dose unitaire est comprise entre 1E8 et 3E14 génomes vecteurs.

3. Composition destinée à être utilisée selon la revendication 1, dans laquelle la dose unitaire est comprise entre 1E9 et 3E13 génomes vecteurs.

4. Composition destinée à être utilisée selon la revendication 1, dans laquelle la dose unitaire est comprise entre 1E10 et 1E13 génomes vecteurs.

5. Composition destinée à être utilisée selon la revendication 1, dans laquelle la dose unitaire est comprise entre 2E12 et 6E12 génomes vecteurs.

6. Composition destinée à être utilisée selon l'une quelconque des revendications 1 à 5, le sujet étant un humain.

7. Composition destinée à être utilisée selon l'une quelconque des revendications 1 à 6, l'affection ou la maladie oculaire étant la néovascularisation choroïdienne ou la DMLA humide.

8. Composition destinée à être utilisée selon l'une quelconque des revendications 1 à 7, dans laquelle l'administration de la composition entraîne une réduction du pourcentage de lésions de grade IV d'au moins 5 %, ou d'au moins 10 %, par rapport à un véhicule témoin, tel que mesuré par la photographie en couleur du fond d'œil.

9. Composition destinée à être utilisée selon l'une quelconque des revendications précédentes, dans laquelle la dose unitaire : (a) a un volume qui n'est pas supérieur à 100 L ; ou (b) a un volume qui n'est pas supérieur à 50 L.

10. Composition destinée à être utilisée selon l'une quelconque des revendications précédentes, le sujet étant sensible au ranibizumab, et/ou au bevacizumab, et/ou au sVEGFR-1.

11. Composition destinée à être utilisée selon l'une quelconque des revendications précédentes, le sujet ayant été prétraité par du ranibizumab ou par du bevacizumab.

12. Composition destinée à être utilisée selon l'une quelconque des revendications précédentes, dans laquelle : (a) l'administration par injection n'a lieu pas plus d'une fois en au moins 2 ans ou pas plus d'une fois en au moins 5 ans ; ou (b) l'administration est une administration unique.

13. Composition destinée à être utilisée selon l'une quelconque des revendications précédentes, la composition pharmaceutique étant une suspension.

14. Composition destinée à être utilisée selon la revendication 13, comprenant en outre : (a) l'agitation de la suspension pour assurer une distribution uniforme avant l'étape d'administration ; et/ou (b) le réchauffement de la suspension à température ambiante avant l'étape d'administration.

15. Composition destinée à être utilisée selon la revendication 13 ou la revendication 14, dans laquelle la suspension comprend en outre : (a) un tensioactif, éventuellement dans laquelle le tensioactif est choisi parmi les polysorbates, le dodécyl sulfate de sodium, le lauryl sulfate de sodium, l'oxyde d'amine de lauryl diméthyl, les alcools polyéthoxylés, le polyoxyéthylène sorbitan, l'octoxynol, le Brij, le pluronic et l'huile de ricin polyoxyle ; et/ou (b) du phénol, du mannitol, du sorbitol ou du chlorure de sodium.

16. Composition destinée à être utilisée selon l'une quelconque des revendications précédentes, comprenant en outre l'administration d'une solution antibiotique, par exemple une solution antibiotique comprenant de la ciprofloxacine, ou une pommade au sulfate d'atropine après l'injection.

17. Composition destinée à être utilisée selon l'une quelconque des revendications précédentes, dans laquelle le variant rAAV2 comprend un acide nucléique codant pour la séquence de SEQ ID NO : 9 et la séquence de SEQ ID NO : 10.
